# EUROPEAN PATENT APPLICATION

(11) **EP 2 213 733 A2**
(43) Date of publication of application: **04.08.2010**
(21) Application number: 10156328.6
(22) Date of filing: 24.05.2007
(51) Int. Cl.: C12N 9/64, A61K 38/48, A61P 7/04

(54) **Factor IX analogues having prolonged in vivo half life**

(30) Priority: 24.05.2006 EP 06114492
(62) Divisional of application: 07765281.6
(71) Applicant: Novo Nordisk Health Care AG, 8050 Zürich (CH)
(72) Inventor: Oestergaard, Henrik, 3650 Oelstykke (DK); Olsen, Ole Hvilsted, 2700 Broenshoej (DK); Stennicke, Henning, 2980 Kokkedal (DK); Steenstrup, Thomas, 2820 Gentofte (DK)
(74) Representative: Nilsson, Karin Norvin

(57) **Abstract**

The invention is related to FIX analogues which have an increased circulation time in the blood stream before activation compared to that that of native FIX (and a week after injection to a patient retains at least about 5% of the FIX activity compared to the initial activity peak value reached after injection). The claimed FIX analogues comprise an inserted cysteine residue which has been further modified by conjugation with a chemical group increasing the molecular weight of the FIX analogue.

## Description

### FIELD OF THE INVENTION

The present invention is related to certain blood coagulation FIX analogues and derivatives with a prolonged circulation time in the blood stream compared to native FIX.

### BACKGROUND OF THE INVENTION

Factor IXa (FIXa) is a trypsin-like serine protease that serves a key role in haemostasis by generating, as part of the Xase complex, most of the factor Xa required to support proper thrombin formation during coagulation (reviewed in (Hoffman and Monroe, III 2001)). Congenital deficiency of factor IXa activity is the cause of the X-linked bleeding disorder haemophilia B affecting approximately 1:100,000 males. These haemophilia patients are currently treated by replacement theraphy with either recombinant or plasma-derived coagulation FIX. However, recommendations are now moving from traditional on-demand treatment towards prophylaxis. Current prophylaxis therapy requires multiple dosing a week, but for optimal plasma levels and efficacy, once-daily injections would be superior. Due to the practical and economical limitations associated with daily administrations, this is not currently an option for the patients. Thus, there is a clear need for a long-acting recombinant factor IX product.

WO 2006005058 relates to conjugates of a factor IX moiety and one or more water-soluble polymers with a size from 5-150 kDa. WO 2006018204 relates to modified vitamin k-dependent polypeptides comprising a modified activation peptide. WO2004101740 relates to a chimeric protein comprising at least one clotting factor and at least a portion of an immunoglobulin constant region. WO 2005001025 relates to a chimeric monomer-dimer hybrid protein wherein said protein comprises a first polypeptide chain comprising a portion of an immunoglobulin constant region and a biologically active molecule and a second polypeptide chain comprising a portion of an immunoglobulin constant region without the biologically active molecule. US 2006040856 relates to conjugates between factor IX and PEG moieties linked via an intact glycosyl linking group interposed between and covalently attached to the peptide and the modifying group.

SE 9501285A discloses a process for the *in vitro* production of appropriately folded, biologically active disulfide-crosslinked proteins using a mixture of a protein disulfide oxidoreductase (e.g. protein disulfide isomerase (PDI)), a glutaredoxin and a redox buffer. The reference is focused on cysteines involved in intramolecular disulfide bonds.

WO 2006/134173 relates to method for selective reduction and derivatization of recombinantly prepared engineered proteins comprising at least one non-native cysteine including Factor IX polypeptides.

It is an object of the invention to provide modified FIX analogues or derivatives with a prolonged circulation time in the blood stream compared to native FIX while retaining sufficient biological activity to support blood clotting.

### SUMMARY OF THE INVENTION

In one aspect the present invention relates to a FIX analogue with a prolonged circulatory half-life, wherein at least one of the natural amino residues in position 44, 46, 47, 50, 53, 57, 66, 67, 68, 70, 72, 74, 80, 84, 87, 89, 90, 91, 94, 100, 101, 102, 103, 104, 105, 106, 108, 113, 116, 119, 120, 121, 123, 125, 129, 138, 140, 141, 142, 146-180, 185, 186, 188, 189, 201, 202, 203, 224, 225, 228, 239, 240, 241, 243, 247, 249, 252, 257, 260, 261, 262, 263, 265, 277, 280, 314, 316, 318, 321, 341, 372, 374, 391, 392, 406, 410, 413 or 415 is substituted for a cysteine amino acid residue conjugated with a chemical group increasing the molecular weight of the FIX polypeptide.

In a preferred embodiment the FIX analogue has at least one of the natural amino residues in position 44, 46, 47, 50, 53, 57, 66, 67, 68, 70, 72, 74, 80 or 84 is substituted for a cysteine amino acid residue conjugated with a chemical group increasing the molecular weight of the FIX polypeptide.

In another preferred embodiment the FIX analogue has at least one of the natural amino residues in position 87, 89, 90, 91, 94, 100, 101, 102, 103, 104, 105, 106, 108, 113, 116, 119, 120, 121, 123, 125, 129, 138, 140, 141 or 142, is substituted for a cysteine amino acid residue conjugated with a chemical group increasing the molecular weight of the FIX polypeptide.

In another preferred embodiment the FIX analogue has at least one of the natural amino residues in position 185, 186, 188, 189, 201, 202, 203, 224, 225, 228, 239, 240, 241, 243, 247, 249, 252, 257, 260, 261, 262, 263, 265, 277, 280, 314, 316, 318, 321, 341, 372, 374, 391, 392, 406, 410, 413 or 415, is substituted for a cysteine amino acid residue conjugated with a chemical group increasing the molecular weight of the FIX polypeptide

In another preferred embodiment the FIX analogue has at least one of the natural amino residues in position 146-180, substituted for a cysteine amino acid residue conjugated with a chemical group increasing the molecular weight of the FIX polypeptide.

It is to be understood that since position 146-180 corresponds to the activation peptide of FIX, which is removed upon activation, a FIX analogue, wherein at least one of the natural amino residues is substituted for a cysteine amino acid residue in a position selected from 146-180, which cysteine is conjugated with a chemical group increasing the molecular weight of the FIX polypeptide, this chemical group will not be present on the analogue following activation.

In another preferred embodiment the FIX analogue has at least one of the natural amino residues selected from positions 155, 156, 157, 158, 159, 160, 161, 162, 163, 164, 167, 168,169, 170, 171, 172, 173, 174, 175, 176 177, is substituted for a cysteine amino acid residue conjugated with a chemical group increasing the molecular weight of the FIX polypeptide.

In another preferred embodiment the FIX analogue has at least one of the natural amino residues selected form positions 160, 161, 162, 163, 164, 165 and 166, is substituted for a cysteine amino acid residue conjugated with a chemical group increasing the molecular weight of the FIX polypeptide.

In another preferred embodiment the FIX analogue has amino acid residue E162 substituted for a cysteine amino acid residue conjugated with a chemical group increasing the molecular weight of the FIX polypeptide.

Another aspect of the present invention relates to a FIX analogue with a prolonged circulatory half-life, which has at least one cysteine residue appended to the C-terminal T415, said cysteine being conjugated with a chemical group increasing the molecular weight of the FIX polypeptide.

In preferred embodiments of the present invention, the chemical group conjugated to the non-native cysteine residue polyethylene glycols (PEG). In a further preferred embodiment the polyethyleneglycol has an average molecular weight of in the range of 500-100,000, such as 1000-75,000, or 2,000-60,000.

In preferred FIX analogues has a circulatory half-life of at least 1.5 times that of wild-type FIX.

In preferred FIX analogues of the present invention the analogue, when measured in a clotting assay, has a biological activity of at least 20% of wild-type FIX.

Another aspect of the present invention relates to a method for preparing a FIX analogue, comprising the steps of a) selectively reducing an engineered FIX polypeptide comprising at least one non-native cysteine in a position selected from the group of position 44, 46, 47, 50, 53, 57, 66, 67, 68, 70, 72, 74, 80, 84, 87, 89, 90, 91, 94, 100, 101, 102, 103, 104, 105, 106, 108, 113, 116, 119, 120, 121, 123, 125, 129, 138, 140, 141, 142, 146-180, 185, 186, 188, 189, 201, 202, 203, 224, 225, 228, 239, 240, 241, 243, 247, 249, 252, 257, 260, 261, 262, 263, 265, 277, 280, 314, 316, 318, 321, 341, 372, 374, 391, 392, 406, 410, 413 or 415, conjugated through a disulfide bridge to a low-molecular weight thiol (RS-CYS), by allowing the low-molecular weight thiol-conjugated FIX to react with a mixture comprising a redox buffer and b) a simultaneous or subsequent step of conjugating at least one of the selectively reduced cysteine (HS-Cys) moieties with a chemical group.

In a preferred embodiment the method comprises a redox buffer comprising a mixture comprising reduced and oxidized glutathione. In a further preferred embodiment the redox buffer further comprises a glutaredoxin.

Another aspect of the present invention relates to a method for preparing a FIX analogue, comprising the steps of a) selectively reducing an engineered FIX polypeptide comprising at least one non-native cysteine in a position selected from the group of position 44, 46, 47, 50, 53, 57, 66, 67, 68, 70, 72, 74, 80, 84, 87, 89, 90, 91, 94, 100, 101, 102, 103, 104, 105, 106, 108, 113, 116, 119, 120, 121, 123, 125, 129, 138, 140, 141, 142, 146-180, 185, 186, 188, 189, 201, 202, 203, 224, 225, 228, 239, 240, 241, 243, 247, 249, 252, 257, 260, 261, 262, 263, 265, 277, 280, 314, 316, 318, 321, 341, 372, 374, 391, 392, 406, 410, 413 or 415, conjugated through a disulfide bridge to a low-molecular weight thiol (RS-CYS), by allowing the low-molecular weight thiol-conjugated FIX to react with a mixture comprising a triarylphosphine-3,3',3"-trisulfonic acid compound and b) a simultaneous or subsequent step of conjugating at least one of the selectively reduced cysteine (HS-Cys) moieties with a chemical group.

In preferred embodiments of the method of the present invention the chemical group is selected from polyethylene glycols (PEG), in particular one having an average molecular weight of in the range of 500-100,000, such as 1000-75,000, or 2,000-60,000.

Another aspect of the invention relates to a pharmaceutical formulation for the treatment of a haemophilia patient comprising a therapeutically effective amount of a FIX analogue according to the invention together with a pharmaceutically acceptable carrier.

Another aspect of the present invention relates to a method of treating of a haemophilia patient comprising administering to the patient a therapeutically effective amount of a FIX analogue according to the invention together with a pharmaceutically acceptable carrier.

In a preferred embodiment the method according comprises at least once weekly treatment. In another preferred embodiment the treatment comprises only once weekly treatment.

### DETAILED DESCRIPTION OF THE INVENTION

FIX is a vitamin K-dependent coagulation factor with structural similarities to factor VII, prothrombin, factor X, and protein C. The circulating zymogen form, which has a plasma half-life of about 18-30 hours, consists of 415 amino acids divided into four distinct domains comprising an N-terminal γ-carboxyglutamic acid rich (Gla) domain, two EGF domains, and a C-terminal trypsin-like serine protease domain. Activation of FIX occurs by limited proteolysis at Arg¹⁴⁵-Ala¹⁴⁶ and Arg¹⁸⁰-Val¹⁸¹ releasing a 35-aa fragment, the so-called activation peptide (Schmidt and Bajaj 2003). The activation peptide is heavily glycosylated containing two N-linked and up to four O-linked glycans.

Covalent modification, e.g. by PEGylation or lipid attachment, has been successfully applied on several protein-based pharmaceutics to improve their pharmacokinetic and pharmacodynamic profiles. Conjugation via native or engineered cysteines provides an attractive means of site-specific modification due to the rarity of this amino acid on the surface of proteins as well as the high selectivity afforded by the thiol-coupling chemistry. In practice, however, this approach is often complicated by the fact that introduced cysteines are found as mixed disulfides with low-molecular weight (LMW) thiols such as cysteine and glutathione preventing subsequent modification.

Thus, there is a need for methods in which mixed disulfides of such cysteines and low-molecular weight thiols can be chemically reduced with preservation of the native disulfide bonds.

The human factor IX gene was isolated and expressed in mammalian cells (K. Kurachi and E. W. Davie. Isolation and characterization of a cDNA coding for human factor IX. PNAS. 79 (21 I):6461-6464, 1982; K. H. Choo, K. G. Gould, D. J. Rees, and G. G. Brownlee. Molecular cloning of the gene for human anti-haemophilic factor IX. Nature 299 (5879):178-180, 1982; R. J. Kaufman, L. C. Wasley, B. C. Furie, B. Furie, and C. B. Shoemaker. Expression, purification, and characterization of recombinant gamma-carboxylated factor IX synthesized in Chinese hamster ovary cells. J.Biol.Chem. 261 (21):9622-9628, 1986.) and the amino acid sequence was deduced from cDNA.

The present treatment of haemophilia B with FIX normally includes around two weekly injections supplemented with injections on a need basis, e.g. before tooth extractions or surgery. FIX circulates as an inactive proform and is only converted in the active form FIXa when a bleeding is to be arrested. Thus one way to accomplish a prophylactic FIX treatment based on e.g. one weekly injection is to increase the circulation time of FIX in the blood stream of the patient. In this way there will always be a certain level of zymogen FIX ready to be activated to ensure normal blood clotting conditions in the patient at any time.

The FIX analogues according to the present invention are modified by substituting one ore more of the natural amino acid residues in FIX with a Cys residue.

Identification of positions in FIX suitable for the introduction of cysteine residues:
The amino acid positions being modified will preferably be taken from the pool of residues having a relative side-chain surface accessibility >50%. Surface accessibilities for the heavy chain and EGF2 domain were calculated from published crystallographic data (1RFN, Hopfner et al, 1999), while calculations on the EGF1 domain were based on a homology model built from the crystal structure of porcine FIXa (1PFX, Brandstetter et al, 1995). In addition, selected residues (residues 155-177) in the activation peptide, for which no structural information is available, were considered important. In a recent publication it was shown that this segment of the activation peptide can be deleted without compromising the biological activity of FIX (Begbie et al. 2005). All relevant positions in human FIX are listed in Table 1.

**Table 1. Relevant positions for site-specific modification of FIX. 'ASA1' is the accessibility (in Å²) of the whole amino acid residue, 'ASA2' is the accessibility (in Å²) of the side chain, and 'Rel. ASA' is the fractional accessibility of the side chain. The sequence numbering follows chymotrypsin and is identical to the numbering in the crystal structure of porcine FIXa (1PFX.pdb). Side chains marked with asterisk (*) or a minus (-) are hypothesised to be important for binding to the FVIIa/TF complex (Chen, C.W. et. al. Thromb Haemost. 2002 ;88 :74-82.) or to FVIIIa (Autin, L. et. al. J. Thromb. Haem. 2005, 3: 2044-56), respectively.**

| **FIX EGF1** | | | | | |
|---|---|---|---|---|---|
| **Res. No.** | **Residue** | **ASA1** | **ASA2** | **Rel ASA** | **Mark** |
| 44 | GLN | 98.9 | 89.62 | 0.65 | |
| 45 | TYR | 127.61 | 102.77 | 0.56 | * |
| 46 | VAL | 92.03 | 79.54 | 0.73 | |
| 47 | ASP | 120.51 | 105.86 | 0.91 | |
| 50 | GLN | 94.29 | 81.75 | 0.56 | |
| 52 | GLU | 137.29 | 121.94 | 0.85 | * |
| 53 | SER | 110.6 | 71.75 | 0.89 | |
| 54 | ASN | 78.81 | 62.97 | 0.54 | * |
| 57 | LEU | 94.46 | 85.04 | 0.57 | |
| 59 | GLY | 64.16 | 47.95 | 0.89 | * |
| 61 | SER | 57.29 | 47.74 | 0.58 | * |
| 66 | ILE | 128.4 | 117.58 | 0.77 | |
| 67 | ASN | 116.62 | 99.47 | 0.86 | |
| 68 | SER | 65.52 | 63.95 | 0.84 | |
| 70 | GLU | 104.08 | 98.67 | 0.71 | |
| 72 | TRP | 143.49 | 135.57 | 0.63 | |
| 74 | PRO | 109.62 | 91.25 | 0.73 | * |
| 80 | LYS | 191.42 | 178.01 | 0.99 | |
| 84 | LEU | 103.29 | 98.38 | 0.65 | |

| **FIX EGF2** | | | | | |
|---|---|---|---|---|---|
| **Res. No.** | **Residue** | **ASA1** | **ASA2** | **Rel ASA** | **Mark** |
| 87 | THR | 83.57 | 76.41 | 0.66 | |
| 89 | ASN | 122.26 | 99.36 | 0.8 | |
| 90 | ILE | 84 | 79.82 | 0.53 | |
| 91 | LYS | 128.1 | 96.73 | 0.58 | |
| 94 | ARG | 151.51 | 138.14 | 0.69 | |
| 100 | LYS | 118.47 | 114.68 | 0.67 | |
| 101 | ASN | 78.92 | 63.6 | 0.56 | |
| 102 | SER | 83 | 53.18 | 0.63 | |
| 103 | ALA | 88.66 | 47.67 | 0.7 | |
| 104 | ASP | 88.46 | 74.73 | 0.62 | |
| 105 | ASN | 108.19 | 99.74 | 0.86 | |
| 106 | LYS | 131.06 | 126.34 | 0.68 | |
| 108 | VAL | 75.49 | 69.94 | 0.59 | |
| 113 | GLU | 101.37 | 87.96 | 0.58 | |
| 116 | ARG | 136.07 | 129.02 | 0.64 | |
| 119 | GLU | 137.93 | 126.99 | 0.82 | |
| 120 | ASN | 113.58 | 86.88 | 0.72 | |
| 121 | GLN | 84.29 | 83 | 0.57 | |
| 123 | SER | 52.41 | 52.41 | 0.57 | |
| 125 | GLU | 76.19 | 76.19 | 0.55 | |
| 129 | PRO | 127.15 | 91.63 | 0.8 | |
| 138 | SER | 99.87 | 72.52 | 0.79 | |
| 140 | THR | 78.22 | 62.84 | 0.6 | |
| 141 | SER | 124.17 | 90.36 | 1.02 | |
| 142 | LYS | 243.38 | 231.32 | 0.95 | |

| **FIX Heavy chain** | | | | | |
|---|---|---|---|---|---|
| **Res. No.** | **Residue** | **ASA1** | **ASA2** | **Rel ASA** | **Mark** |
| 185 | GLU | 104.18 | 102.68 | 0.73 | |
| 186 | ASP | 93.02 | 81.46 | 0.74 | |
| 188 | LYS | 124.37 | 123.07 | 0.67 | |
| 189 | PRO | 62.18 | 58.27 | 0.51 | |
| 201 | LYS | 174.2 | 136.05 | 0.73 | |
| 202 | VAL | 73.97 | 68.06 | 0.63 | |
| 203 | ASP | 103.23 | 96.57 | 0.79 | |
| 224 | GLU | 110.06 | 99.47 | 0.71 | |
| 225 | THR | 136.54 | 115.79 | 1.02 | |
| 228 | LYS | 158.34 | 146.18 | 0.82 | |
| 239 | GLU | 111.9 | 87.55 | 0.58 | |
| 240 | GLU | 105.36 | 95.43 | 0.66 | |
| 241 | THR | 93.62 | 82.06 | 0.72 | |
| 243 | HIS | 160.28 | 127.85 | 0.82 | |
| 247 | LYS | 147.15 | 133.67 | 0.73 | |
| 249 | ASN | 62.4 | 62.4 | 0.54 | |
| 252 | ARG | 120.71 | 108.77 | 0.57 | |
| 257 | HIS | 134.87 | 98.77 | 0.65 | |
| 260 | ASN | 71.4 | 63.12 | 0.56 | |
| 261 | ALA | 62.45 | 44.52 | 0.68 | |
| 262 | ALA | 94.53 | 49.72 | 0.76 | |
| 263 | ILE | 141.89 | 110.3 | 0.74 | |
| 265 | LYS | 100.02 | 99.84 | 0.54 | |
| 277 | GLU | 104.36 | 104.36 | 0.73 | |
| 280 | VAL | 102.37 | 98.01 | 0.82 | |
| 292 | ASP | 72.62 | 59.01 | 0.52 | - |
| 293 | LYS | 115.6 | 115.56 | 0.67 | - |
| 294 | GLU | 107.3 | 101.01 | 0.68 | - |
| 301 | LYS | 146.99 | 124.63 | 0.65 | - |
| 314 | PHE | 120.75 | 120.08 | 0.72 | |
| 316 | LYS | 165.63 | 138.79 | 0.76 | |
| 318 | ARG | 170.14 | 155.69 | 0.8 | |
| 321 | LEU | 98.82 | 95.81 | 0.61 | |
| 327 | ARG | 120.09 | 116.48 | 0.55 | * |
| 332 | ASP | 79.51 | 60.87 | 0.55 | - |
| 333 | ARG | 148.18 | 139.33 | 0.67 | - |
| 338 | ARG | 188.9 | 160.87 | 0.76 | - |
| 341 | LYS | 220.48 | 183.47 | 0.99 | |
| 342 | PHE | 118.35 | 106 | 0.59 | - |
| 343 | THR | 92.32 | 77.52 | 0.68 | - |
| 346 | ASN | 61.25 | 59.08 | 0.51 | - |
| 354 | HIS | 106.42 | 105.15 | 0.63 | * |
| 355 | GLU | 100.57 | 89.47 | 0.69 | * |
| 372 | GLU | 93.79 | 75.65 | 0.53 | |
| 374 | GLU | 140.35 | 102.57 | 0.72 | |
| 391 | MET | 108.83 | 100.48 | 0.67 | |
| 392 | LYS | 160.88 | 145.95 | 0.81 | |
| 406 | ASN | 104.27 | 98.78 | 0.79 | |
| 410 | GLU | 117.19 | 97.59 | 0.68 | |
| 413 | LYS | 153.75 | 134.03 | 0.75 | |
| 415 | THR | 201.09 | 182.54 | 0.95 | |

| **FIX Activation Peptide** | | | | | |
|---|---|---|---|---|---|
| **Res. No.** | **Residue** | | | | |
| 155 | Tyr | | | | |
| 156 | Val | | | | |
| 157 | Asn | | | | |
| 158 | Ser | | | | |
| 159 | Thr | | | | |
| 160 | Glu | | | | |
| 161 | Ala | | | | |
| 162 | Glu | | | | |
| 163 | Thr | | | | |
| 164 | Ile | | | | |
| 165 | Leu | | | | |
| 166 | Asp | | | | |
| 167 | Asn | | | | |
| 168 | Ile | | | | |
| 169 | Thr | | | | |
| 170 | Gln | | | | |
| 171 | Ser | | | | |
| 172 | Thr | | | | |
| 173 | Gln | | | | |
| 174 | Ser | | | | |
| 175 | Phe | | | | |
| 176 | Asn | | | | |
| 177 | Asp | | | | |

The FIX analogues of the present invention have a prolonged circulatory half-life as compared to wild-type FIX. The circulatory half.life can be measured by methods known to the skilled person such as .e.g. described in McCarthy et al Thromb Haemost. 2002 May;87(5):824-30.

In preferred embodiments the circulatory half-life of the FIX analogues of the present invention is at least 1.5 times that of wild-type FIX, such as at least 1.6, e.g at least 1.7, such as at least 1.8, e.g. at least 1.9, such as at least 2.0, e.g at least 2.2, such as at least 2.4 times that of wild-type FIX when measured in the same assay or model.

The FIX analogues of the present invention furthermore retains sufficient biological activity to support clot formation in vivo. The biological activity may be measured by methods known to the skilled person such as e.g. described in McCarthy et al Thromb Haemost. 2002 May;87(5):824-30.

In preferred embodiments the clotting activity of the FIX analogues of the present invention is at least 15 %, such as at least 20%, e.g. at least 25 %, such as at least 30 %, e.g. at least 35 %, such as at least 40%, e.g. at least 50 %, such as at least 60%, e.g. at least 70 % of the clotting activity of wild-type FIX when measured in the same assay.

### Mutating, expressing & purifying

The FIX analogues may be produced by means of recombinant nucleic acid techniques. In general, a cloned human nucleic acid sequence is modified to encode the desired FIX analogue and is then inserted into an expression vector, which is in turn transformed or transfected into host cells. Higher eukaryotic cells, in particular cultured mammalian cells, are preferred as host cells.

The amino acid sequence alterations may be accomplished by a variety of techniques. Modification of the nucleic acid sequence may be by site-specific mutagenesis. Techniques for site-specific mutagenesis are well known in the art and are described in, for example, Zoller and Smith (DNA 3:479-488, 1984) or "Splicing by extension overlap", Horton et al., Gene 77, 1989, pp. 61-68. Thus, using the nucleotide and amino acid sequences of FIX, one may introduce the alteration(s) of choice. Likewise, procedures for preparing a DNA construct using polymerase chain reaction using specific primers are well known to persons skilled in the art (cf. PCR Protocols, 1990, Academic Press, San Diego, California, USA).

The nucleic acid construct encoding the FIX analogue of the invention may be of genomic or cDNA origin, for instance obtained by preparing a genomic or cDNA library and screening for DNA sequences coding for all or part of FIX by hybridization using synthetic oligonucleotide probes in accordance with standard techniques (cf. Sambrook et al., Molecular Cloning: A Laboratory Manual, 2nd. Ed. Cold Spring Harbor Labora-tory, Cold Spring Harbor, New York, 1989).

The nucleic acid construct encoding the FIX polypeptide analogue may also be prepared synthetically by established standard methods, e.g. the phosphoamidite method described by Beaucage and Caruthers, Tetrahedron Letters 22 (1981), 1859 - 1869, or the method described by Matthes et al., EMBO Journal 3 (1984), 801 - 805. According to the phosphoamidite method, oligonucleotides are synthesised, e.g. in an automatic DNA synthesiser, purified, annealed, ligated and cloned in suitable vectors. The DNA sequences encoding the human FIX polypeptides may also be prepared by polymerase chain reaction using specific primers, for instance as described in US 4,683,202, Saiki et al., Science 239 (1988), 487 - 491, or Sambrook et al., supra.

Furthermore, the nucleic acid construct may be of mixed synthetic and genomic, mixed synthetic and cDNA or mixed genomic and cDNA origin prepared by ligating fragments of syn-thetic, genomic or cDNA origin (as appropriate), the fragments corresponding to various parts of the entire nucleic acid construct, in accordance with standard techniques.

The DNA sequences encoding the FIX polypeptides are usually inserted into a recombinant vector which may be any vector, which may conveniently be subjected to recombinant DNA procedures, and the choice of vector will often depend on the host cell into which it is to be introduced. Thus, the vector may be an autonomously replicating vector, i.e. a vector, which exists as an extrachromosomal entity, the replication of which is independent of chromosomal replication, e.g. a plasmid. Alternatively, the vector may be one which, when introduced into a host cell, is integrated into the host cell genome and replicated together with the chromosome(s) into which it has been integrated.

The vector is preferably an expression vector in which the DNA sequence encoding the FIX analogue is operably linked to additional segments required for transcription of the DNA. In general, the expression vector is derived from plasmid or viral DNA, or may contain elements of both. The term, "operably linked" indicates that the segments are arranged so that they function in concert for their intended purposes, e.g. transcription initiates in a promoter and proceeds through the DNA sequence coding for the polypeptide.

Expression vectors for use in expressing FIX analogues will comprise a promoter capable of directing the transcription of a cloned gene or cDNA. The promoter may be any DNA sequence, which shows transcriptional activity in the host cell of choice and may be derived from genes encoding proteins either homologous or heterologous to the host cell.

Examples of suitable promoters for directing the transcription of the DNA encoding the FIX analogues in mammalian cells are the SV40 promoter (Subramani et al., Mol. Cell Biol. 1 (1981), 854 -864), the MT-1 (metallothionein gene) promoter (Palmiter et al., Science 222 (1983), 809 - 814), the CMV promoter (Boshart et al., Cell 41:521-530, 1985) or the adenovirus 2 major late promoter (Kaufman and Sharp, Mol. Cell. Biol, 2:1304-1319,1982).

The DNA sequences encoding the FIX analogue may also, if necessary, be operably connected to a suitable terminator, such as the human growth hormone terminator (Palmiter et al., Science 222, 1983, pp. 809-814) or the TPI1 (Alber and Kawasaki, J. Mol. Appl. Gen. 1, 1982, pp. 419-434) or ADH3 (McKnight et al., The EMBO J. 4, 1985, pp. 2093-2099) terminators. Expression vectors may also contain a set of RNA splice sites located downstream from the promoter and upstream from the insertion site for the FIX sequence itself. Preferred RNA splice sites may be obtained from adenovirus and/or immunoglobulin genes. Also contained in the expression vectors is a polyadenylation signal located downstream of the insertion site. Particularly preferred polyadenylation signals include the early or late polyadenylation signal from SV40 (Kaufman and Sharp, ibid.), the polyadenylation signal from the adenovirus 5 Elb region, the human growth hormone gene terminator (DeNoto et al. Nucl. Acids Res. 9:3719-3730, 1981) or the polyadenylation signal from the human FIX gene. The expression vectors may also include a noncoding viral leader sequence, such as the adenovirus 2 tripartite leader, located between the promoter and the RNA splice sites; and enhancer sequences, such as the SV40 enhancer.

To direct the FIX analogue of the present invention into the secretory pathway of the host cells, a secretory signal sequence (also known as a leader sequence, prepro sequence or pre sequence) may be provided in the recombinant vector. The secretory signal sequence is joined to the DNA sequences encoding the FIX analogues in the correct reading frame. Secretory signal sequences are commonly positioned 5' to the DNA sequence encoding the peptide. The secretory signal sequence may be that, normally associated with the protein or may be from a gene encoding another secreted protein.

The procedures used to ligate the DNA sequences coding for the FIX analogues, the promoter and optionally the terminator and/or secretory signal sequence, respectively, and to insert them into suitable vectors containing the information necessary for replication, are well known to persons skilled in the art (cf., for instance, Sambrook et al., Molecular Cloning: A Laboratory Manual, Cold Spring Harbor, New York, 1989).

Methods of transfecting mammalian cells and expressing DNA sequences introduced in the cells are described in e.g. Kaufman and Sharp, J. Mol. Biol. 159 (1982), 601 - 621; Southern and Berg, J. Mol. Appl. Genet. 1 (1982), 327 - 341; Loyter et al., Proc. Natl. Acad. Sci. USA 79 (1982), 422 - 426; Wigler et al., Cell 14 (1978), 725; Corsaro and Pearson, Somatic Cell Genetics 7 (1981), 603, Graham and van der Eb, Virology 52 (1973), 456; and Neumann et al., EMBO J. 1 (1982), 841 - 845.

Cloned DNA sequences are introduced into cultured mammalian cells by, for example, calcium phosphate-mediated transfection (Wigler et al., Cell 14:725-732, 1978; Corsaro and Pearson, Somatic Cell Genetics 7:603-616, 1981; Graham and Van der Eb, Virology 52d:456-467, 1973) or electroporation (Neumann et al., EMBO J. 1:841-845, 1982). To identify and select cells that express the exogenous DNA, a gene that confers a selectable phenotype (a selectable marker) is generally introduced into cells along with the gene or cDNA of interest. Preferred selectable markers include genes that confer resistance to drugs such as neomycin, hygromycin, and methotrexate. The selectable marker may be an amplifiable selectable marker. A preferred amplifiable selectable marker is a dihydrofolate reductase (DHFR) sequence. Selectable markers are reviewed by Thilly (Mammalian Cell Technology, Butterworth Publishers, Stoneham, MA, incorporated herein by reference). The person skilled in the art will easily be able to choose suitable selectable markers.

Selectable markers may be introduced into the cell on a separate plasmid at the same time as the gene of interest, or they may be introduced on the same plasmid. Constructs of this type are known in the art (for example, Levinson and Simonsen, U.S. 4,713,339). It may also be advantageous to add additional DNA, known as "carrier DNA," to the mixture that is introduced into the cells.

After the cells have taken up the DNA, they are grown in an appropriate growth me-dium, typically 1-2 days, to begin expressing the gene of interest. As used herein the term "appropriate growth medium" means a medium containing nutrients and other components required for the growth of cells and the expression of the FIX analogues. Media generally include a carbon source, a nitrogen source, essential amino acids, essential sugars, vitamins, salts, phospholipids, protein and growth factors. Drug selection is then applied to select for the growth of cells that are expressing the selectable marker in a stable fashion. For cells that have been transfected with an amplifiable selectable marker the drug concentration may be increased to select for an increased copy number of the cloned sequences, thereby in-creasing expression levels. Clones of stably transfected cells are then screened for expression of the FIX analogue.

Examples of mammalian cell lines for use in the present invention are the COS-1 (ATCC CRL 1650), baby hamster kidney (BHK) and 293 (ATCC CRL 1573; Graham et al., J. Gen. Virol. 36:59-72, 1977) cell lines. A preferred BHK cell line is the tk- ts13 BHK cell line (Waechter and Baserga, Proc. Natl. Acad. Sci. USA 79:1106-1110, 1982, incorporated herein by reference), hereinafter referred to as BHK 570 cells. The BHK 570 cell line has been deposited with the American Type Culture Collection, 12301 Parklawn Dr., Rockville, Md. 20852, under ATCC accession number CRL 10314. A tk- ts13 BHK cell line is also available from the ATCC under accession number CRL 1632. In addition, a number of other cell lines may be used within the present invention, including Rat Hep I (Rat hepatoma; ATCC CRL 1600), Rat Hep II (Rat hepatoma; ATCC CRL 1548), TCMK (ATCC CCL 139), Human lung (ATCC HB 8065), NCTC 1469 (ATCC CCL 9.1), CHO (ATCC CCL 61) and CHO-DUKX cells (Urlaub and Chasin, Proc. Natl. Acad. Sci. USA 77:4216-4220, 1980).

FIX analogues of the invention are recovered from cell culture medium and can then be purified by a variety of procedures known in the art including, but not limited to, chromatography (e.g., ion exchange, affinity, hydrophobic, chromatofocusing, and size exclusion), electrophoretic procedures (e.g., preparative isoelectric focusing (IEF), differential solubility (e.g., ammonium sulfate precipitation), or extraction (see, e.g., Protein Purification, J.-C. Janson and Lars Ryden, editors, VCH Publishers, New York, 1989). Preferably, they may be purified by affinity chromatography on an anti-FIX antibody column. Additional purification may be achieved by conventional chemical purification means, such as high performance liquid chromatography. Other methods of purification are known in the art, and may be applied to the purification of the novel FIX polypeptides described herein (see, for example, Scopes, R., Protein Purification, Springer-Verlag, N.Y., 1982).

For therapeutic purposes it is preferred that the FIX analogue is purified to at least about 90 to 95% homogeneity, preferably to at least about 98% homogeneity. Purity may be assessed by e.g. gel electrophoresis and amino-terminal amino acid sequencing.

### Selective reduction

In view of the above-mentioned obstacles with respect to chemical conjugation via thiol groups of cysteines not involved in intramolecular disulfide bonds of proteins prepared by recombinant techniques, the present invention provides the use of a defined redox buffer mixture (e.g. in combination with a thiol-disulfide redox catalyst) or a triarylphosphine to selectively reduce the mixed disulfide bond between low-molecular weight thiols and an engineered protein comprising at least one non-native cysteine, e.g. a coagulation factor or a protein of the trypsin family of serine proteases, with such engineered or native cysteines. Following selective reducing of the mixed disulfide, the free cysteine can then be modified by conjugation using thiol-coupling chemistry on the protein as known to people skilled in the art.

Chemical conjugation via engineered or native cysteines offers the choice of targeted modification of proteins yielding a single homogenous product. However, in cases where the cysteine is conjugated to a low-molecular weight thiol and the protein contains labile intramolecular disulfide bonds this strategy is not feasible. The present invention enables selective removal of the low-molecular weight thiol moiety preparing the liberated cysteine for subsequent chemical modification.

Hence, one aspect of the present invention relates to a method for selective reduction of an engineered FIX, i.e. comprising at least one non-native cysteine, said FIX comprising one or more cysteine moieties conjugated through a disulfide bridge to a low-molecular weight thiol (RS-Cys), said moiety/moieties not being involved in intramolecular S-S bridges (Cys-S-S-Cys) when the protein is in its active form, the method comprising the step of allowing the low-molecular weight thiol-conjugated protein to react with a mixture comprising a redox buffer.

Another aspect of the present invention relates to a method for selective reduction of an engineered FIX, i.e. comprising at least one non-native cysteine, said protein comprising one or more cysteine moieties conjugated through a disulfide bridge to a low-molecular weight thiol (RS-Cys), said moiety/moieties not being involved in intramolecular S-S bridges (Cys-S-S-Cys) when the protein is in its active form, the method comprising the step of allowing the low-molecular weight thiol-conjugated protein to react with a mixture comprising a triarylphosphine reducing agent.

The term "selectively reduced" refers to the fact that a predominant portion, e.g. a fraction of 60% or more, or 80% or more, such as 90% or more, of the cysteine moieties conjugated through a disulfide bridge to a low-molecular weight thiol are reduced to liberate a cysteine moiety with a thiol group which is ready for conjugation with other groups, whereas predominant portion, e.g. 60% or more, or 80% or more, such as 90% or more, of other disulfide bonds (typically intramolecular disulfide bonds) are preserved so that the biological activity of the engineered protein is substantially preserved.

### (A) Redox buffer

As mentioned above, the present invention i.a. provides a method for selective reduction of an engineered protein comprising at least one non-native cysteine, e.g. a coagulation factor or a protein of the trypsin-family of serine proteases. The protein in question comprises one or more cysteine moieties conjugated through a disulfide bridge to a low-molecular weight thiol (RS-Cys), said moiety/moieties not being involved in intramolecular S-S bridges (Cys-S-S-Cys) when the protein is in its active form, the method comprising the step of allowing the low-molecular weight thiol-conjugated protein to react with a mixture comprising a redox buffer.

When used herein, the term "redox buffer" is intended to mean a thiol/disulfide redox pair in a ratio that is sufficiently reducing to disrupt the protein-low-molecular weight thiol mixed disulfide(s) (RS-Cys) and at the same time sufficiently oxidizing to preserve the integrity of the native disulfide bonds in the protein.

Preferably, the redox buffer comprises a low molecular weight thiol/disulfide redox pair. By the term "low molecular weight" is meant that the thiol-form of the redox pair has a molecular weight of at the most 500 g/mol. Illustrative examples of such redox pairs are the ones selected from (i) reduced and oxidized glutathione and (ii) reduced and oxidized γ-glutamylcysteine, (iii) reduced and oxidized cysteinylglycine, (iv) reduced and oxidized cysteine, (v) reduced and oxidized N-acetylcysteine, (vi) cysteamine, and (vii) dihydrolipoamide/lipoamide, preferably from (i) reduced and oxidized glutathione.

The optimal redox conditions can be determined by performing a redox titration of the protein as known to the person skilled in the art. See Gilbert (1995).

In one embodiment, the redox buffer is a redox pair of reduced and oxidized glutathione, and the concentration of the reduced glutathione is in the range of 0-100 mM, and the ratio between reduced glutathione and oxidized glutathione is in the range of 2-200.

In another embodiment, the redox buffer is a redox pair of reduced and oxidized glutathione, and the concentration of the reduced glutathione is in the range of 0-100 mM, e.g. 0.01-50 mM, and the concentration of the oxidized glutathione is in the range of 0-5 mM, e.g. 0.001-5 mM. For Factor IX polypeptides, the concentration of the reduced glutathione is preferably in the range of 0-5 mM, e.g. 0.01-2 mM, and the concentration of the oxidized glutathione is in the range of 0.001-2 mM, e.g. 0.001-0.200 mM.

Since glutathione and other low molecular-weight thiols are generally poor reductants/oxidants in terms of reaction kinetics, a thiol/disulfide redox catalyst is most preferably included in the mixture in conjunction with the redox buffer in order to enhance the rate of the reaction.

Suitable thiol/disulfide redox catalysts to be included in the mixture include dithiol-type and monothiol-type glutaredoxins. Glutaredoxins and their functions are generally described in Fernandes et al. (2004). Useful examples of glutaredoxins are those selected from Grx1, Grx2 or Grx3 from *Escherichia coli* (Holmgren et al., 1995), Grx1p, Grx2p, Grx3p, Grx4p, and Grx5p from *Saccharomyces cerevisiae* (Luikenhuis et al. 1998; Rodriguez-Manzaneque et al., 1999; Grant, 2001), Grx1 and Grx2 from *Homo sapiens* (Padilla et al. 1995; Lundberg et al., 2001), and variants hereof. Variants include, but is not restricted to, dithiol-type glutaredoxins in which the C-terminal cysteine in the CXXC motif has been replaced by another amino acid typically serine (see Yang et al., 1998).

The redox catalyst (in particular a glutaredoxin) is preferably used in a concentration of 0.001-20 µM.

It is preferred that the mixture does not comprise a protein disulfide isomerase (PDI).

The redox buffer may further comprise other components such as salts, pH buffers, etc., and the method of the invention may be conducted at any temperature which is suitable for the protein in question, e.g. a temperature in the range of from -5°C to 50°C, such as in the range of from 0°C to 37°C, of course dependent on the stability of the protein under the given conditions.

### (B) Triarylphosphine reducing agent

As mentioned above, the present invention also provides a method for selective reduction of an engineered protein comprising at least one non-native cysteine, e.g. a coagulation factor or a protein of the trypsin-family of serine proteases. The protein in question comprises one or more cysteine moieties conjugated through a disulfide bridge to a low-molecular weight thiol (RS-Cys), said moiety/moieties not being involved in intramolecular S-S bridges (Cys-S-S-Cys) when the protein is in its active form, the method comprising the step of allowing the low-molecular weight thiol-conjugated protein to react with a triarylphosphine reducing agent.

The term "triarylphosphine reducing agent" is intended to mean a triarylphosphine optionally substituted with one or more substituents.

The aryl groups of the triarylphosphine reducing agent are preferably selected from phenyl, naphthyl, 1,2,3,4-tetrahydronaphthyl, anthracyl, phenanthracyl, pyrenyl, benzopyrenyl, fluorenyl and xanthenyl, in particular phenyl, and in currently selected embodiments, the aryl groups are preferably identical. In the currently most interesting embodiment, all three aryl groups are phenyl. Examples of substituents, which may be present in the aryl groups, in particular phenyl groups, are typically those selected from sulfonic acid, carboxylic acid, C₁₋₆-alkyl, C₁₋₆-alkoxy, and C₁₋₆-alkoxy-C₁₋₆-alkyl, or C₃₋₆-alkylene (representing a ring with two neighboring aryl carbon atoms) or C₂₋₆-alkyleneoxy (representing a ring with two neighboring aryl carbon atoms) or C₁₋₄-alkylene-oxy-C₁₋₄-alkylen (representing a ring with two neighboring aryl carbon atoms).

In the currently most interesting embodiments, at least one aryl (e.g. phenyl) has at least one substituent selected from sulfonic acid and carboxylic acid, in particular sulfonic acid; such substituent preferably being arranged in the meta position relative to the bond to the phosphor atom.

Preferably, all three aryl groups have a sulfonic acid substituent, e.g. all three aryl groups have a sulfonic acid substituent and at least one further substituent, in particular at least a substituent in the para-position relative to the bond to the phosphor atom, in particular an oxygen substituent in this para-position.

It is currently believed that the aryl groups of preferred reducing agents do not have any substituents in the ortho-position relative to the bond to the phosphor atom.

The term "C₁₋₆-alkyl" is intended to encompass linear or branched saturated hydrocarbon residues which have 1-6 carbon atoms. Particular examples are methyl, ethyl, n-propyl, isopropyl, n-butyl, isobutyl, sec-butyl, tert-butyl, n-pentyl, isopentyl, n-hexyl, etc. Similarly, the term "C₁₋₄-alkyl" encompasses linear or branched saturated hydrocarbon residues which have 1-4 carbon atoms. The terms "C₁₋₆-alkylene", "C₂₋₆-alkylene", etc. represent the biradicals corresponding to "C₁₋₆-alkyl", "C₂₋₆-alkyl", respectively.

Suitable triarylphosphine reducing agents are those having a useful balance between reduction potential and steric hinderance. The chemical nature of the triarylphosphine reducing agent is dictated by its ability to cleave the protein-low-molecular weight thiol mixed disulfide (RS-Cys) while preserving the integrity of the native disulfide bonds in the protein. Currently very interesting compounds are triarylphosphine trisulfonic acids, such as triphenylphosphine-3,3',3"-trisulfonic acid and analogues hereof. Illustrative examples hereof are triarylphosphine reducing agents selected from triphenylphosphine-3,3',3"-trisulfonic acid and analogues thereof, e.g. one of those selected from the compounds 9-11 below:

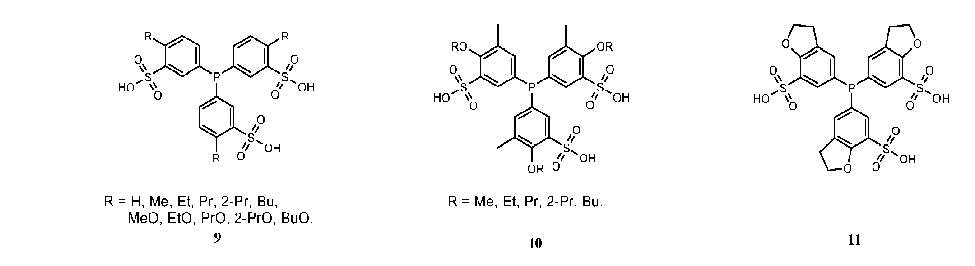

The triarylphosphine reducing agent is preferably used in a concentration of 0.001-100 mM, such as 0.01-50 mM or 0.1-25 mM.

In one interesting embodiment, the triarylphosphine reducing agent is immobilized to a solid support. This will facilitate the easy separation of the reducing agent from the protein. In general, triarylphosphine reducing agent, such as compounds 9-11, may be immobilized by means known to the person skilled in the art, e.g. by introducing a linker group in one of the aryl groups. The triarylphosphine reagent 12 is an example of a linkable variant of 1.

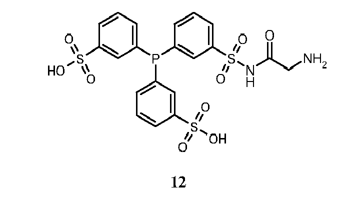

The reaction is typically conducted at a temperature in the range of 0-40°C, such as at ambient temperature, for a period of from 5 seconds to several days, as the case may be. The reaction may be followed by HPLC in order to confirm the conversion. The solvent is preferably an aqueous buffer, optionally including co-solvents such as DMSO or DMF. The solvent may also comprise salts, e.g. calcium salts.

### Conjugation

One important purpose of the selective reduction methods described above is to liberate a cysteine group which can be used for attachment (conjugation) of a chemical group, e.g. a non-polypeptide moiety.

Hence, in one important embodiment, the method further comprises the simultaneous and/or subsequent step of conjugating at least one of the selectively reduced cysteine (HS-Cys) moiety/moieties with a chemical group.

It should be understood that the conjugation of the at least one selectively reduced cysteine moieties with a chemical group may be conducted simultaneously, i.e. by addition of one or more reagents leading to the conjugation to the mixture comprising the redox buffer, or in a subsequent step, e.g. after purification and/or isolation of the selectively reduced protein.

In one embodiment, the chemical group is a protractor group, i.e. a group which upon conjugation to the protein (e.g. Factor IX polypeptide) increases the circulation half-life of said protein or polypeptide, when compared to the un-modified protein or polypeptide. The specific principle behind the protractive effect may be caused by increased size, shielding of peptide sequences that can be recognized by peptidases or antibodies, or masking of glycanes in such way that they are not recognized by glycan specific receptores present in e.g. the liver or on macrophages, preventing or decreasing clearance. The protractive effect of the protractor group can e.g. also be caused by binding to blood components sush as albumin, or unspecific adhesion to vascular tissue. The conjugated glycoprotein should substantially preserve its biological activity.

In one embodiment of the invention the protractor group is selected from the group consisting of:
(a) A low molecular organic charged radical (15-1,000 Da), which may contain one or more carboxylic acids, amines sulfonic acids, phosphonic acids, or combination thereof.
(b) A low molecular (15-1,000 Da) neutral hydrophilic molecule, such as cyclodextrin, or a polyethylene chain which may optionally branched.
(c) A low molecular (15-1,000 Da) hydrophobic molecule such as a fatty acid or cholic acid or derivatives thereof.
(d) Polyethyleneglycol with an average molecular weight of 2,000-60,000 Da.
(e) A well defined precision polymer such as a dendrimer with an exact molecular mass ranging from 700 to 20,000 Da, or more preferable between 700-10,000 Da.
(f) A substantially non-immunogenic polypeptide such as albumin or an antibody or part of an antibody optionally containing an Fc-domain.
(g) A high molecular weight organic polymer such as dextran.

In another embodiment of the invention the protractor group is selected from the group consisting of dendrimers, polyalkylene oxide (PAO), including polyalkylene glycol (PAG), such as polyethylene glycol (PEG) and polypropylene glycol (PPG), branched PEGs, polyvinyl alcohol (PVA), polycarboxylate, poly-vinylpyrolidone, polyethylene-co-maleic acid anhydride, polystyrene-co-maleic acid anhydride, and dextran, including carboxymethyl-dextran. In one particularly interesting embodiment of the invention, the protractor group is a PEG group.

The term "branched polymer", or interchangebly "dendritic polymer", "dendrimer" or "dendritic structure" means an organic polymer assembled from a selection of monomer building blocks of which, some contains branches.

In one embodiment of the invention the protractor group is a selected from the goup consisting of serum protein binding-ligands, such as compounds which bind to albumin, like fatty acids, C5-C24 fatty acid, aliphatic diacid (e.g. C5-C24). Other examples of protractor groups includes small organic molecules containing moieties that under physiological conditions alters charge properties, such as carboxylic acids or amines, or neutral substituents that prevent glycan specific recognition such as smaller alkyl substituents (e.g., C1-C5 alkyl). In one embodiment of the invention the protractor group is albumin.

In one embodiment, the chemical group is a non-polypeptide.

In one interesting embodiment, the chemical group is a polyethyleneglycol (PEG), in particular one having an average molecular weight of in the range of 500-100,000, such as 1,000-75,000, or 2,000-60,000.

Conjugation can be conducted as disclosed in WO 02/077218 A1 and WO 01/58935 A2.

Particularly interesting is the use of PEG as a chemical group for conjugation with the protein. The term "polyethylene glycol" or "PEG" means a polyethylene glycol compound or a derivative thereof, with or without coupling agents, coupling or activating moeities (e.g., with thiol, triflate, tresylate, azirdine, oxirane, pyridyldithio, vinyl sulfone, or preferably with a maleimide moiety). Compounds such as maleimido monomethoxy PEG are exemplary of activated PEG compounds of the invention.

PEG is a suitable polymer molecule, since it has only few reactive groups capable of cross-linking compared to polysaccharides such as dextran. In particular, monofunctional PEG, e.g. methoxypolyethylene glycol (mPEG), is of interest since its coupling chemistry is relatively simple (only one reactive group is available for conjugating with attachment groups on the polypeptide). Consequently, the risk of cross-linking is eliminated, the resulting polypeptide conjugates are more homogeneous and the reaction of the polymer molecules with the polypeptide is easier to control.

To effect covalent attachment of the polymer molecule(s) to the polypeptide, the hydroxyl end groups of the polymer molecule are provided in activated form, i.e. with reactive functional groups. Suitable activated polymer molecules are commercially available, e.g. from Shearwater Corp., Huntsville, Ala., USA, or from PolyMASC Pharmaceuticals plc, UK. Alternatively, the polymer molecules can be activated by conventional methods known in the art, e.g. as disclosed in WO 90/13540. Specific examples of activated linear or branched polymer molecules for use in the present invention are described in the Shearwater Corp. 1997 and 2000 Catalogs (Functionalized Biocompatible Polymers for Research and pharmaceuticals, Polyethylene Glycol and Derivatives, incorporated herein by reference). Specific examples of activated PEG polymers include the following linear PEGs: NHS-PEG (e.g. SPA-PEG, SSPA-PEG, SBA-PEG, SS-PEG, SSA-PEG, SC-PEG, SG-PEG, and SCM-PEG), and NOR-PEG), BTC-PEG, EPOX-PEG, NCO-PEG, NPC-PEG, CDI-PEG, ALD-PEG, TRES-PEG, VS-PEG, IODO-PEG, and MAL-PEG, and branched PEGs such as PEG2-NHS and those disclosed in U.S. Pat. No. 5,932,462 and U.S. Pat. No. 5,643,575, both of which are incorporated herein by reference. Furthermore, the following publications, incorporated herein by reference, disclose useful polymer molecules and/or PEGylation chemistries: U.S. Pat. No. 5,824,778, U.S. Pat. No. 5,476,653, WO 97/32607, EP 229,108, EP 402,378, U.S. Pat. No. 4,902,502, U.S. Pat. No. 5,281,698, U.S. Pat. No. 5,122,614, U.S. Pat. No. 5,219,564, WO 92/16555, WO 94/04193, WO 94/14758, WO 94/17039, WO 94/18247, WO 94/28024, WO 95/00162, WO 95/11924, WO 95/13090, WO 95/33490, WO 96/00080, WO 97/18832, WO 98/41562, WO 98/48837, WO 99/32134, WO 99/32139, WO 99/32140, WO 96/40791, WO 98/32466, WO 95/06058, EP 439 508, WO 97/03106, WO 96/21469, WO 95/13312, EP 921 131, U.S. Pat. No. 5,736,625, WO 98/05363, EP 809 996, U.S. Pat. No. 5,629,384, WO 96/41813, WO 96/07670, U.S. Pat. No. 5,473,034, U.S. Pat. No. 5,516,673, EP 605 963, U.S. Pat. No. 5,382,657, EP 510 356, EP 400 472, EP 183 503 and EP 154 316.

The conjugation of the polypeptide and the activated polymer molecules is conducted by use of any conventional method, e.g. as described in the following references (which also describe suitable methods for activation of polymer molecules): R. F. Taylor, (1991), "Protein immobilisation. Fundamental and applications", Marcel Dekker, N.Y.; S. S. Wong, (1992), "Chemistry of Protein Conjugation and Crosslinking", CRC Press, Boca Raton; G. T. Hermanson et al., (1993), "Immobilized Affinity Ligand Techniques", Academic Press, N.Y.). The skilled person will be aware that the activation method and/or conjugation chemistry to be used depends on the attachment group(s) of the polypeptide

(examples of which are given further above), as well as the functional groups of the polymer (e.g. being amine, hydroxyl, carboxyl, aldehyde, sulfydryl, succinimidyl, maleimide, vinysulfone or haloacetate). The PEGylation may be directed towards conjugation to all available attachment groups on the polypeptide (i.e. such attachment groups that are exposed at the surface of the polypeptide) or may be directed towards one or more specific attachment groups, e.g. the N-terminal amino group (U.S. Pat. No. 5,985,265). Furthermore, the conjugation may be achieved in one step or in a stepwise manner (e.g. as described in WO 99/55377).

It will be understood that the PEGylation is designed so as to produce the optimal molecule with respect to the number of PEG molecules attached, the size and form of such molecules (e.g. whether they are linear or branched), and where in the polypeptide such molecules are attached. The molecular weight of the polymer to be used will be chosen taking into consideration the desired effect to be achieved. For instance, if the primary purpose of the conjugation is to achieve a conjugate having a high molecular weight and larger size (e.g. to reduce renal clearance), one may choose to conjugate either one or a few high molecular weight polymer molecules or a number of polymer molecules with a smaller molecular weight to obtain the desired effect. In one embodiment, several polymer molecules with a lower molecular weight is used. This is also the case if a high degree of epitope shielding is desired. In such cases, 2-8 polymers with a molecular weight of e.g. about 5,000 Da, such as 3-6 such polymers, may for example be used. As the examples below illustrate, it may be advantageous to have a larger number of polymer molecules with a lower molecular weight (e.g. 4-6 with a M_{W} of 5,000) compared to a smaller number of polymer molecules with a higher molecular weight (e.g. 1-3 with a MW of 12,000-20,000) in terms of improving the functional in vivo half-life of the polypeptide conjugate, even where the total molecular weight of the attached polymer molecules in the two cases is the same or similar. It is believed that the presence of a larger number of smaller polymer molecules provides the polypeptide with a larger diameter or apparent size than e.g. a single yet larger polymer molecule, at least when the polymer molecules are relatively uniformly distributed on the polypeptide surface.

It has further been found that advantageous results are obtained when the apparent size (also referred to as the "apparent molecular weight" or "apparent mass") of at least a major portion of the conjugate of the invention is at least about 50 kDa, such as at least about 55 kDa, such as at least about 60 kDa, e.g. at least about 66 kDa. This is believed to be due to the fact that renal clearance is substantially eliminated for conjugates having a sufficiently large apparent size. In the present context, the "apparent size" of a protein conjugate or Factor IX polypeptide is determined by the SDS-PAGE method.

Furthermore, it has been reported that excessive polymer conjugation can lead to a loss of activity of the protein (e.g. Factor IX polypeptide) to which the chemical group (e.g. a non-polypeptide moiety) is conjugated (see further below). This problem can be eliminated, e.g., by removal of attachment groups located at the functional site or by reversible blocking the functional site prior to conjugation so that the functional site of the protein is blocked during conjugation. Specifically, the conjugation between the protein and the chemical group (e.g. non-polypeptide moiety) may be conducted under conditions where the functional site of the protein is blocked by a helper molecule e.g. a serine protease inhibitor. Preferably, the helper molecule is one, which specifically recognizes a functional site of the protein, such as a receptor or active-site inhibitor binding to and thus protecting the area around the catalytic triad (preferably defined as amino acid residues within 10 Å of any atom in the catalytic triad).

Alternatively, the helper molecule may be an antibody, in particular a monoclonal antibody recognizing the protein (e.g. Factor IX polypeptide). In particular, the helper molecule may be a neutralizing monoclonal antibody.

The protein is preferably to interact with the helper molecule before effecting conjugation. (Often it is even advantageous to use the same helper molecule (e.g. an inhibitor) as the one used in the steps where mixed disulfides are reduced.) This ensures that the functional site of the protein (e.g. Factor IX polypeptide) is shielded or protected and consequently unavailable for derivatization by the chemical group (e.g. non-polypeptide moiety) such, as a polymer.

Following its elution from the helper molecule, the conjugate of the chemical group and the protein can be recovered with at least a partially preserved functional site.

### Formulations and administration

In another aspect the present invention is related to a pharmaceutical formulation comprising a FIX analogue in a dried form, whereto the physician or the patient adds solvents and/or diluents prior to use. By "dried form" is intended the liquid pharmaceutical composition or formulation is dried either by freeze drying (i.e., lyophilization; see, for example, Williams and Polli (1984) J. Parenteral Sci. Technol. 38:48-59), spray drying (see Masters (1991) in Spray-Drying Handbook (5th ed; Longman Scientific and Technical, Essez, U.K.), pp. 491-676; Broadhead et al. (1992) Drug Devel. Ind. Pharm. 18:1169-1206; and Mumenthaler et al. (1994) Pharm. Res. 11:12-20), or air drying

(Carpenter and Crowe (1988) Cryobiology 25:459-470; and Roser (1991) Biopharm. 4:47-53).

In a further aspect the invention relates to a pharmaceutical formulation comprising an aqueous solution of a FIX analogue and a buffer, wherein the FIX analogue is present in a concentration from 0.01 mg/ml or above, and wherein said formulation has a pH from about 2.0 to about 10.0.

In a further embodiment of the invention the buffer is selected from the group consisting of sodium acetate, sodium carbonate, citrate, glycylglycine, histidine, glycine, lysine, arginine, sodium dihydrogen phosphate, disodium hydrogen phosphate, sodium phosphate, and tris(hydroxymethyl)-aminomethan, bicine, tricine, malic acid, succinate, maleic acid, fumaric acid, tartaric acid, aspartic acid or mixtures thereof. Each one of these specific buffers constitutes an alternative embodiment of the invention.

In a further embodiment of the invention the formulation further comprises a pharmaceutically acceptable preservative. In a further embodiment of the invention the preservative is selected from the group consisting of phenol, o-cresol, m-cresol, p-cresol, methyl p-hydroxybenzoate, propyl p-hydroxybenzoate, 2-phenoxyethanol, butyl p-hydroxybenzoate, 2-phenylethanol, benzyl alcohol, chlorobutanol, and thiomerosal, bronopol, benzoic acid, imidurea, chlorohexidine, sodium dehydroacetate, chlorocresol, ethyl p-hydroxybenzoate, benzethonium chloride, chlorphenesine (3p-chlorphenoxypropane-1,2-diol) or mixtures thereof. In a further embodiment of the invention the preservative is present in a concentration from 0.1 mg/ml to 20 mg/ml. In a further embodiment of the invention the preservative is present in a concentration from 0.1 mg/ml to 5 mg/ml. In a further embodiment of the invention the preservative is present in a concentration from 5 mg/ml to 10 mg/ml. In a further embodiment of the invention the preservative is present in a concentration from 10 mg/ml to 20 mg/ml. Each one of these specific preservatives constitutes an alternative embodiment of the invention. The use of a preservative in pharmaceutical compositions is well-known to the skilled person. For convenience reference is made to Remington: The Science and Practice of Pharmacy, 19th edition, 1995.

In a further embodiment of the invention the formulation further comprises an isotonic agent. In a further embodiment of the invention the isotonic agent is selected from the group consisting of a salt (e.g. sodium chloride), a sugar or sugar alcohol, an amino acid (e.g. L-glycine, L-histidine, arginine, lysine, isoleucine, aspartic acid, tryptophan, threonine),
an alditol (e.g. glycerol (glycerine), 1,2-propanediol (propyleneglycol), 1,3-propanediol, 1,3-butanediol) polyethyleneglycol (e.g. PEG400), or mixtures thereof. Any sugar such as mono-, di-, or polysaccharides, or water-soluble glucans, including for example fructose, glucose, mannose, sorbose, xylose, maltose, lactose, sucrose, trehalose, dextran, pullulan, dextrin, cyclodextrin, soluble starch, hydroxyethyl starch and carboxymethylcellulose-Na may be used. In one embodiment the sugar additive is sucrose. Sugar alcohol is defined as a C4-C8 hydrocarbon having at least one -OH group and includes, for example, mannitol, sorbitol, inositol, galactitol, dulcitol, xylitol, and arabitol. In one embodiment the sugar alcohol additive is mannitol. The sugars or sugar alcohols mentioned above may be used individually or in combination. There is no fixed limit to the amount used, as long as the sugar or sugar alcohol is soluble in the liquid preparation and does not adversely effect the stabilizing effects achieved using the methods of the invention. In one embodiment, the sugar or sugar alcohol concentration is between about 1 mg/ml and about 150 mg/ml. In a further embodiment of the invention the isotonic agent is present in a concentration from 1 mg/ml to 50 mg/ml. In a further embodiment of the invention the isotonic agent is present in a concentration from 1 mg/ml to 7 mg/ml. In a further embodiment of the invention the isotonic agent is present in a concentration from 8 mg/ml to 24 mg/ml. In a further embodiment of the invention the isotonic agent is present in a concentration from 25 mg/ml to 50 mg/ml. Each one of these specific isotonic agents constitutes an alternative embodiment of the invention. The use of an isotonic agent in pharmaceutical compositions is well-known to the skilled person. For convenience reference is made to Remington: The Science and Practice of Pharmacy, 19th edition, 1995.

In a further embodiment of the invention the formulation further comprises a chelating agent. In a further embodiment of the invention the chelating agent is selected from salts of ethylenediaminetetraacetic acid (EDTA), citric acid, and aspartic acid, and mixtures thereof. In a further embodiment of the invention the chelating agent is present in a concentration from 0.1mg/ml to 5mg/ml. In a further embodiment of the invention the chelating agent is present in a concentration from 0.1mg/ml to 2mg/ml. In a further embodiment of the invention the chelating agent is present in a concentration from 2mg/ml to 5mg/ml. Each one of these specific chelating agents constitutes an alternative embodiment of the invention. The use of a chelating agent in pharmaceutical compositions is well-known to the skilled person. For convenience reference is made to Remington: The Science and Practice of Pharmacy, 19th edition, 1995.

In a further embodiment of the invention the formulation further comprises a stabilizer. The use of a stabilizer in pharmaceutical compositions is well-known to the skilled person. For convenience reference is made to Remington: The Science and Practice of Pharmacy, 19th edition, 1995.

The pharmaceutical compositions of the invention may further comprise an amount of an amino acid base sufficient to decrease aggregate formation by the polypeptide during storage of the composition. By "amino acid base" is intended an amino acid or a combination of amino acids, where any given amino acid is present either in its free base form or in its salt form. Where a combination of amino acids is used, all of the amino acids may be present in their free base forms, all may be present in their salt forms, or some may be present in their free base forms while others are present in their salt forms. In one embodiment, amino acids to use in preparing the compositions of the invention are those carrying a charged side chain, such as arginine, lysine, aspartic acid, and glutamic acid. Any stereoisomer (i.e., L, D, or DL isomer) of a particular amino acid (e.g. glycine, methionine, histidine, imidazole, arginine, lysine, isoleucine, aspartic acid, tryptophan, threonine and mixtures thereof) or combinations of these stereoisomers, may be present in the pharmaceutical compositions of the invention so long as the particular amino acid is present either in its free base form or its salt form. In one embodiment the L-stereoisomer is used. Compositions of the invention may also be formulated with analogues of these amino acids. By "amino acid analogue" is intended a derivative of the naturally occurring amino acid that brings about the desired effect of decreasing aggregate formation by the polypeptide during storage of the liquid pharmaceutical compositions of the invention. Suitable arginine analogues include, for example, aminoguanidine, ornithine and N-monoethyl L-arginine, suitable methionine analogues include ethionine and buthionine and suitable cysteine analogues include S-methyl-L cysteine. As with the other amino acids, the amino acid analogues are incorporated into the compositions in either their free base form or their salt form. In a further embodiment of the invention the amino acids or amino acid analogues are used in a concentration, which is sufficient to prevent or delay aggregation of the protein.

In a further embodiment of the invention methionine (or other sulphuric amino acids or amino acid analogous) may be added to inhibit oxidation of methionine residues to methionine sulfoxide when the polypeptide acting as the therapeutic agent is a polypeptide comprising at least one methionine residue susceptible to such oxidation. By "inhibit" is intended minimal accumulation of methionine oxidized species over time. Inhibiting methionine oxidation results in greater retention of the polypeptide in its proper molecular form. Any stereoisomer of methionine (L, D, or DL isomer) or combinations thereof can be used. The amount to be added should be an amount sufficient to inhibit oxidation of the methionine residues such that the amount of methionine sulfoxide is acceptable to regulatory agencies. Typically, this means that the composition contains no more than about 10% to about 30% methionine sulfoxide. Generally, this can be achieved by adding methionine such that the ratio of methionine added to methionine residues ranges from about 1:1 to about 1000:1, such as 10:1 to about 100:1.

In a further embodiment of the invention the formulation further comprises a stabilizer selected from the group of high molecular weight polymers or low molecular compounds. In a further embodiment of the invention the stabilizer is selected from polyethylene glycol (e.g. PEG 3350), polyvinyl alcohol (PVA), polyvinylpyrrolidone, carboxy-/hydroxycellulose or derivates thereof (e.g. HPC, HPC-SL, HPC-L and HPMC), cyclodextrins, sulphur-containing substances as monothioglycerol, thioglycolic acid and 2-methylthioethanol, and different salts (e.g. sodium chloride). Each one of these specific stabilizers constitutes an alternative embodiment of the invention.

The pharmaceutical compositions may also comprise additional stabilizing agents, which further enhance stability of a therapeutically active polypeptide therein. Stabilizing agents of particular interest to the present invention include, but are not limited to, methionine and EDTA, which protect the polypeptide against methionine oxidation, and a nonionic surfactant, which protects the polypeptide against aggregation associated with freeze-thawing or mechanical shearing.

In a further embodiment of the invention the formulation comprises a surfactant. The surfactant may be a detergent, ethoxylated castor oil, polyglycolyzed glycerides, acetylated monoglycerides, sorbitan fatty acid esters, polyoxypropylene-polyoxyethylene block polymers (eg. poloxamers such as Pluronic^{®} F68, poloxamer 188 and 407, Triton X-100 ), polyoxyethylene sorbitan fatty acid esters, polyoxyethylene and polyethylene derivatives such as alkylated and alkoxylated derivatives (tweens, e.g. Tween-20, Tween-40, Tween-80 and Brij-35), monoglycerides or ethoxylated derivatives thereof, diglycerides or polyoxyethylene derivatives thereof, alcohols, glycerol, lectins and phospholipids (eg. phosphatidyl serine, phosphatidyl choline, phosphatidyl ethanolamine, phosphatidyl inositol, diphosphatidyl glycerol and sphingomyelin), derivates of phospholipids (eg. dipalmitoyl phosphatidic acid) and lysophospholipids (eg. palmitoyl lysophosphatidyl-L-serine and 1-acyl-sn-glycero-3-phosphate esters of ethanolamine, choline, serine or threonine) and alkyl, alkoxyl (alkyl ester), alkoxy (alkyl ether)- derivatives of lysophosphatidyl and phosphatidylcholines, e.g. lauroyl and myristoyl derivatives of lysophosphatidylcholine, dipalmitoylphosphatidylcholine, and modifications of the polar head group, that is cholines, ethanolamines, phosphatidic acid, serines, threonines, glycerol, inositol, and the positively charged DODAC, DOTMA, DCP, BISHOP, lysophosphatidylserine and lysophosphatidylthreonine, and glycerophospholipids (eg. cephalins), glyceroglycolipids (eg. galactopyransoide), sphingoglycolipids (eg. ceramides, gangliosides), dodecylphosphocholine, hen egg lysolecithin, fusidic acid derivatives- (e.g. sodium taurodihydrofusidate etc.), long-chain fatty acids and salts thereof C6-C12 (eg. oleic acid and caprylic acid), acylcarnitines and derivatives, N^{α}-acylated derivatives of lysine, arginine or histidine, or side-chain acylated derivatives of lysine or arginine, N^{α}-acylated derivatives of dipeptides comprising any combination of lysine, arginine or histidine and a neutral or acidic amino acid, N^{α}-acylated derivative of a tripeptide comprising any combination of a neutral amino acid and two charged amino acids, DSS (docusate sodium, CAS registry no [577-11-7]), docusate calcium, CAS registry no [128-49-4]), docusate potassium, CAS registry no [7491-09-0]), SDS (sodium dodecyl sulphate or sodium lauryl sulphate), sodium caprylate, cholic acid or derivatives thereof, bile acids and salts thereof and glycine or taurine conjugates, ursodeoxycholic acid, sodium cholate, sodium deoxycholate, sodium taurocholate, sodium glycocholate, N-Hexadecyl-N,N-dimethyl-3-ammonio-l-propanesulfonate, anionic (alkyl-aryl-sulphonates) monovalent surfactants, zwitterionic surfactants (e.g. N-alkyl-N,N-dimethylammonio-l-propanesulfonates, 3-cholamido-1-propyldimethylammonio-1-propanesulfonate, cationic surfactants (quaternary ammonium bases) (e.g. cetyl-trimethylammonium bromide, cetylpyridinium chloride), non-ionic surfactants (e.g. Dodecyl β-D-glucopyranoside), poloxamines (eg. Tetronic's), which are tetrafunctional block copolymers derived from sequential addition of propylene oxide and ethylene oxide to ethylenediamine, or the surfactant may be selected from the group of imidazoline derivatives, or mixtures thereof. Each one of these specific surfactants constitutes an alternative embodiment of the invention.

The use of a surfactant in pharmaceutical compositions is well-known to the skilled person. For convenience reference is made to Remington: The Science and Practice of Pharmacy, 19th edition, 1995.

It is possible that other ingredients may be present in the pharmaceutical formulation of the present invention. Such additional ingredients may include wetting agents, emulsifiers, antioxidants, bulking agents, tonicity modifiers, chelating agents, metal ions, oleaginous vehicles, proteins (e.g., human serum albumin, gelatine or proteins) and a zwitterion (e.g., an amino acid such as betaine, taurine, arginine, glycine, lysine and histidine). Such additional ingredients, of course, should not adversely affect the overall stability of the pharmaceutical formulation of the present invention.

Parenteral administration may be performed by subcutaneous, intramuscular, intraperitoneal or intravenous injection by means of a syringe, optionally a pen-like syringe. Alternatively, parenteral administration can be performed by means of an infusion pump. A further option is a composition which may be a solution or suspension for the administration of the FIX compound in the form of a nasal or pulmonal spray. As a still further option, the pharmaceutical compositions containing the FIX compound of the invention can also be adapted to transdermal administration, e.g. by needle-free injection or from a patch, optionally an iontophoretic patch, or transmucosal, *e.g*. buccal, administration.

### DEFINITIONS

"Factor IX" or "FIX" as used herein refers to a human plasma Factor IX glycoprotein that is a member of the intrinsic coagulation pathway and is essential to blood coagulation. It is to be understood that this definition includes native as well as recombinant forms of this human plasma Factor IX glycoprotein. Unless otherwise specified or indicated, as used herein factor IX means any functional human factor IX protein molecule in its normal role in coagulation, including any fragment, analogue and derivative thereof.

"Native FIX" is the full length human FIX molecule as shown in SEQ ID NO:1. The numbering of the amino acid residue position is according to SEQ ID NO:1 where the first N-terminal amino acid residue is number 1 and so on.

The terms "analogue" or "analogues", as used herein, is intended to designate Factor FIX having the sequence of SEQ ID NO:1, wherein one or more amino acids of the parent protein have been substituted by another amino acid and/or wherein one or more amino acids of the parent protein have been deleted and/or wherein one or more amino acids have been inserted in protein and/or wherein one or more amino acids have been added to the parent protein. Such addition can take place either at the N-terminal end or at the C-terminal end of the parent protein or both. The "analogue" or "analogues" within this definition still have FIX activity in its activated form. In one embodiment a variant is 70 % identical with the sequence of of SEQ ID NO:1. In one embodiment a variant is 80 % identical with the sequence of of SEQ ID NO:1. In another embodiment a variant is 90 % identical with the sequence of of SEQ ID NO:1. In a further embodiment a variant is 95 % identical with the sequence of of SEQ ID NO:1. As used herein any reference to a specific positions refers to the corresponding position in SEQ ID NO:1.

Unless otherwise specified, factor IX domains include the following amino acid residues: Gla domain being the region from reside Tyr1 to residue Lys43; EGF1 being the region from residue Gln44 to residue Leu84; EGF2 being the region from residue Asp85 to residue Arg145; the Activation Peptide being the region from residue Ala146 to residue Arg180; and the Protease Domain being the region from residue Val181 to Thr414. The light chain refers to the region encompassing the Gla domain, EGF1 and EGF2, while the heavy chain refers to the Protease Domain.

"FIX half-life" refers to the half-life of factor IX in blood circulation, as determined in animals such as mice or in human, as determined by pharmacokinetics by standard procedures known to people skilled in the art.

" FIX activity" or "FIX biological activity" is defined as the ability to function in the coagulation cascade, induce the formation of FXa via interaction with FVIIIa on an activated platelet, and support the formation of a blood clot. The activity may be assessed in vitro by techniques such as clot analysis, as described in e.g. McCarthy et al Thromb Haemost. 2002 May;87(5):824-30, and other techniques known to people skilled in the art.

"Prolonged FIX" means a FIX compound that circulates in a patient for an extended period of time following administration as compared to the native human FIX.

The term "inserted amino residue" is intended to include both a substitution of a natural amino acid residue with another amino acid residue, which is not normally found in that position in the native FIX molecule, and an addition of an amino acid residue to the native human FIX molecule. The addition of an amino acid residue may be either between two existing amino acid residues or at the N- or C-terminal end of the native FIX molecule.

The term "PEGylated FIX" means FIX having a PEG molecule conjugated to the FIX molecule. The term "cysteine-PEGylated FIX" means FIX having a PEG molecule conjugated to a sulfhydryl group of a cysteine introduced in FIX molecule.

The terminology for amino acid substitutions used is as follows. The first letter represents the amino acid residue naturally present at a position of human FVIII. The following number represents the position in human FIX. The second letter represent the different amino acid substituting for (replacing) the natural amino acid. An example is E162C, where a lysine at position 162 of human FIX is replaced by a cysteine.

In the present context the three-letter or one-letter indications of the amino acids have been used in their conventional meaning as indicated in table 2. Unless indicated explicitly, the amino acids mentioned herein are L-amino acids. Further, the left and right ends of an amino acid sequence of a peptide are, respectively, the N- and C-termini unless otherwise specified.

**Table 2: Abbreviations for amino acids:**

| **Amino acid** | **Tree-letter code** | **One-letter code** |
|---|---|---|
| Glycine | Gly | G |
| Proline | Pro | P |
| Alanine | Ala | A |
| Valine | Val | V |
| Leucine | Leu | L |
| Isoleucine | Ile | I |
| Methionine | Met | M |
| Cysteine | Cys | C |
| Phenylalanine | Phe | F |
| Tyrosine | Tyr | Y |
| Tryptophan | Trp | W |
| Histidine | His | H |
| Lysine | Lys | K |
| Arginine | Arg | R |
| Glutamine | Gln | Q |
| Asparagine | Asn | N |
| Glutamic Acid | Glu | E |
| Aspartic Acid | Asp | D |
| Serine | Ser | S |
| Threonine | Thr | T |
| gamma-carboxyglutamic acid | GLA or Xaa | |

The term "low-molecular weight thiol-conjugated (RS-Cys) form" and similar terms are intended to mean that a thiol group of a cysteine of the protein in question is conjugated with a compound having a thiol group, wherein said compound has a molecular weight of less than 500 Da. Examples of such compounds are glutathione, gamma-glutamylcysteine, cysteinylglycine, cysteine, N-acetylcysteine, cysteamine, etc.

The term "active form" refers to the form (or forms) of the protein wherein it is capable of performing a desirable action, e.g. as a catalyst (enzyme), zymogen, or as a co-factor, etc. The "active form" is sometimes referred to as the "correctly folded form".

The protein is generally an "engineered" polypeptide which compared to a native protein includes at least one non-native cysteine. Such "engineered" polypeptides are preferably prepared by recombinant techniques as will be apparent for the person skilled in the art; see also WO 02/077218 A1 and WO 01/58935 A2.

All references, including publications, patent applications, and patents, cited herein are hereby incorporated by reference in their entirety and to the same extent as if each reference were individually and specifically indicated to be incorporated by reference and were set forth in its entirety herein (to the maximum extent permitted by law).

All headings and sub-headings are used herein for convenience only and should not be construed as limiting the invention in any way.

The use of any and all examples, or exemplary language (e.g., "such as") provided herein, is intended merely to better illuminate the invention and does not pose a limitation on the scope of the invention unless otherwise claimed. No language in the specification should be construed as indicating any non-claimed element as essential to the practice of the invention.

The citation and incorporation of patent documents herein is done for convenience only and does not reflect any view of the validity, patentability, and/or enforceability of such patent documents.

This invention includes all modifications and equivalents of the subject matter recited in the claims appended hereto as permitted by applicable law.

Embodiments of the invention:
1. FIX analogue with a prolonged circulatory half-life, wherein at least one of the natural amino residues in position 44, 46, 47, 50, 53, 57, 66, 67, 68, 70, 72, 74, 80, 84, 87, 89, 90, 91, 94, 100, 101, 102, 103, 104, 105, 106, 108, 113, 116, 119, 120, 121, 123, 125, 129, 138, 140, 141, 142, 146-180, 185, 186, 188, 189, 201, 202, 203, 224, 225, 228, 239, 240, 241, 243, 247, 249, 252, 257, 260, 261, 262, 263, 265, 277, 280, 314, 316, 318, 321, 341, 372, 374, 391, 392, 406, 410, 413 or 415 is substituted for a cysteine amino acid residue conjugated with a chemical group increasing the molecular weight of the FIX polypeptide.
2. FIX analogue according to embodiment 1, wherein at least one of the natural amino residues in position 44, 46, 47, 50, 53, 57, 66, 67, 68, 70, 72, 74, 80 or 84 is substituted for a cysteine amino acid residue conjugated with a chemical group increasing the molecular weight of the FIX polypeptide.
3. FIX analogue according to embodiment 1, wherein at least one of the natural amino residues in position 87, 89, 90, 91, 94, 100, 101, 102, 103, 104, 105, 106, 108, 113, 116, 119, 120, 121, 123, 125, 129, 138, 140, 141 or 142, is substituted for a cysteine amino acid residue conjugated with a chemical group increasing the molecular weight of the FIX polypeptide.
4. FIX analogue according to embodiment 1, wherein at least one of the natural amino residues in position 185, 186, 188, 189, 201, 202, 203, 224, 225, 228, 239, 240, 241, 243, 247, 249, 252, 257, 260, 261, 262, 263, 265, 277, 280, 314, 316, 318, 321, 341, 372, 374, 391, 392, 406, 410, 413 or 415, is substituted for a cysteine amino acid residue conjugated with a chemical group increasing the molecular weight of the FIX polypeptide
5. FIX analogue according to embodiment 1, wherein at least one of the natural amino residues in position 146-180, is substituted for a cysteine amino acid residue conjugated with a chemical group increasing the molecular weight of the FIX polypeptide.
6. FIX analogue according to embodiment 1, wherein at least one of the natural amino residues selected from positions 155, 156, 157, 158, 159, 160, 161, 162, 163, 164, 167, 168,169, 170, 171, 172, 173, 174, 175, 176 177, is substituted for a cysteine amino acid residue conjugated with a chemical group increasing the molecular weight of the FIX polypeptide.
7. FIX analogue according to embodiment 1, wherein at least one of the natural amino residues selected form positions 160, 161, 162, 163, 164, 165 and 166, is substituted for a cysteine amino acid residue conjugated with a chemical group increasing the molecular weight of the FIX polypeptide.
8. FIX analogue according to embodiment 7, wherein amino acid residue E162 is substituted for a cysteine amino acid residue conjugated with a chemical group increasing the molecular weight of the FIX polypeptide.
9. FIX analogue with a prolonged circulatory half-life, wherein at least one cysteine residue is appended to the C-terminal T415, said cysteine being conjugated with a chemical group increasing the molecular weight of the FIX polypeptide.
10. FIX analogue according to any of the previous embodiments, wherein the chemical group is selected from polyethylene glycols (PEG).
11. FIX analogue according to embodiment 10, wherein the polyethyleneglycol has an average molecular weight of in the range of 500-100,000, such as 1000-75,000, or 2,000-60,000.
12. FIX analogue according to any of the previous embodiments, wherein the analogue has a circulatory half-life of at least 1.5 times that of wild-type FIX.
13. FIX analogue according to any of the previous embodiments, wherein the analogue, when measured in a clotting assay, has a biological activity of at least 20% of wild-type FIX.
14. A method for preparing a FIX analogue according to any of the previous embodiments, comprising the steps of a) selectively reducing an engineered FIX polypeptide comprising at least one non-native cysteine in a position selected from the group of position 44, 46, 47, 50, 53, 57, 66, 67, 68, 70, 72, 74, 80, 84, 87, 89, 90, 91, 94, 100, 101, 102, 103, 104, 105, 106, 108, 113, 116, 119, 120, 121, 123, 125, 129, 138, 140, 141, 142, 146-180, 185, 186, 188, 189, 201, 202, 203, 224, 225, 228, 239, 240, 241, 243, 247, 249, 252, 257, 260, 261, 262, 263, 265, 277, 280, 314, 316, 318, 321, 341, 372, 374, 391, 392, 406, 410, 413 or 415, conjugated through a disulfide bridge to a low-molecular weight thiol (RS-CYS), by allowing the low-molecular weight thiol-conjugated FIX to react with a mixture comprising a redox buffer and b) a simultaneous or subsequent step of conjugating at least one of the selectively reduced cysteine (HS-Cys) moieties with a chemical group.
15. A method according to embodiment 14, wherein the redox buffer comprises a a mixture comprising reduced and oxidized glutathione and a glutaredoxin.
16. A method for preparing a FIX analogue according to any of embodiments 1-13, comprising the steps of a) selectively reducing an engineered FIX polypeptide comprising at least one non-native cysteine in a position selected from the group of position 44, 46, 47, 50, 53, 57, 66, 67, 68, 70, 72, 74, 80, 84, 87, 89, 90, 91, 94, 100, 101, 102, 103, 104, 105, 106, 108, 113, 116, 119, 120, 121, 123, 125, 129, 138, 140, 141, 142, 146-180, 185, 186, 188, 189, 201, 202, 203, 224, 225, 228, 239, 240, 241, 243, 247, 249, 252, 257, 260, 261, 262, 263, 265, 277, 280, 314, 316, 318, 321, 341, 372, 374, 391, 392, 406, 410, 413 or 415, conjugated through a disulfide bridge to a low-molecular weight thiol (RS-CYS), by allowing the low-molecular weight thiol-conjugated FIX to react with a mixture comprising a triarylphosphine-3,3',3"-trisulfonic acid compound and b) a simultaneous or subsequent step of conjugating at least one of the selectively reduced cysteine (HS-Cys) moieties with a chemical group..
17. A method according to embodiments 14-16, wherein the chemical group is selected from polyethylene glycols (PEG).
18. The method according to embodiment 17, wherein the chemical group is a polyethyleneglycol, in particular one having an average molecular weight of in the range of 500-100,000, such as 1000-75,000, or 2,000-60,000.
19. A pharmaceutical formulation for the treatment of a haemophilia patient comprising a therapeutically effective amount of a FIX analogue according to embodiment 1-13 together with a pharmaceutically acceptable carrier.
20. A method of treating of a haemophilia patient comprising administering to the patient a therapeutically effective amount of a FIX analogue according to any of embodiments 1-13 together with a pharmaceutically acceptable carrier.
21. A method according to embodiment 20, wherein the treatment comprises at least once weekly treatment.
22. A method according to embodiment 21, wherein the treatment comprises only once weekly treatment.

### EXAMPLES

The terminology for amino acid substitutions used in the following examples is as follows. The first letter represents the amino acid naturally present at a position of SEQ ID NO:1. The following number represents the position in SEQ ID NO:1. The second letter represents the different amino acid substituting for the natural amino acid. An example is factor IX E162C, where a glutamic acid at position 162 of SEQ ID No: 1 is replaced by a cysteine.

*Materials* -Reduced and oxidized glutathione (GSH and GSSG, respectively) were purchased from Sigma. PEG5k-maleimide (2E2M0H01), PEG20k-maleimide (2E2M0P01), PEG40k-maleimide (2D3Y0T01) were purchased from Nektar Therapeutics (Huntsville, AL). All other chemicals were of analytical grade or better.

*Concentration determination -* The concentration of GSSG in stock solutions was determined from its absorption at 248 nm using an extinction coefficient of 381 M⁻¹cm⁻¹ (Chau and Nelson, 1991). The concentration of GSH was determined using Ellman's reagent (5,5'-dithiobis(2-nitrobenzoic acid)) and 14150 M⁻¹cm⁻¹ as the molar extinction coefficient of 2-nitro-5-thiobenzoic acid at 412 nm (Riddles et al., 1979).

*Cloning and expression of glutaredoxins -* The DNA coding sequence for *Escherichia coli* glutaredoxin 2 (Grx2) was amplified by PCR using Expand High Fidelity PCR system (Roche Diagnostics Corporation, Indianapolis, IN) according to manufacturer's recommendations and primer pair oHOJ98-f/oHOJ98 introducing flanking *Nde*I and *Xho*I restriction sites (primer sequences are listed in Table 1). Genomic template DNA for the PCR reaction was prepared from E.coli according to published procedure (Grimberg et al., 1989). The purified PCR product was cut with NdeI and XhoI and then ligated into the corresponding sites of pET-24a(+) (Novagen) to give pHOJ294. Since a stop codon was provided by the vector, the gene was equipped with a 3' vector-derived extension encoding a C-terminal LEHHHHHH affinity tag. The correct identity of the cloned sequence was verified by DNA sequencing.

For expression, the 294 plasmid was introduced into chemical competent BL21(DE3) cells (Stratagene, La Jolla, CA). Fresh overnight transformants were inoculated into 500 ml terrific broth ((Sambrook et al., 1989)) and 30 µg/ml kanamycine to an initial OD600 of 0.02. Cultures were grown at 37°C in baffled flasks at 230 rpm to the mid-log phase (OD600 3-4) at which time the temperature was lowered to 25°C and protein expression induced by 0.1 mM isopropyl-β-D-thiogalactopyranoside (ITPG). After overnight expression, cells were harvested by centrifugation, resuspended in 50 ml lysis buffer (50 mM potassium phosphate, 300 mM NaCl, pH 8.0), and lysed by three freeze-thaw cycles. The cleared lysate was loaded onto a 20-ml Ni-NTA Superflow (Qiagen GmbH, Hilden, Germany) column equilibrated with lysis buffer at a flow rate of 5 ml/min. After washing with lysis buffer, bound protein was eluted with a linear gradient from 0-200 mM imidazole in lysis buffer. Peak fractions were pooled, treated with 20mM dithiothreitol for 20 min before extensive dialysis against 50 mM Tris-HCl, 2 mM EDTA, pH 8.0. The protein was stored at -80°C and judged to be >90% pure by SDS-PAGE. Concentration was estimated by absorbance at 280 nm using an extinction coefficients of 21740 M⁻¹cm⁻¹.

### Construction of DNA encoding factor IX and factor IX E162C and factor IX 416C mutants -

Plasmids pHOJ338 and pHOJ358 encoding factor IX E162C and factor IX 416C, respectively, were constructed by QuickChange^{®} Site-Directed Mutagenesis using primer pairs oHOJ137-f/oHOJ137-r and oHOJ155-f/oHOJ155-r (see Table 1) as template according to manufacturer's instructions (Stratagene, La Jolla, CA). The correct identity of all cloned sequences was verified by DNA sequencing.

*Purification of factor IX and variants -* Factor IX and variants are purified as described in Arruda et al. (2001) Blood, 97, 130-138, with the exception that fractions containing factor IX (Cys variants) are pooled, dialyzed against 50 mM HEPES, 100 mM NaCl, 10 mM CaCl₂, pH 7.0 and stored at -80°C.

*Modification of FIX Cys variants with PEG-maleimide -* Selective reduction is carried out by incubating 4.8 mg FIX Cys variant at 30°C for 5 hours in a total volume of 4.4 ml 50 mM HEPES, 100 mM NaCl, 10 mM CaCl₂, pH 7.0 buffer containing 0.5 mM GSH, 20 µM GSSG, and 10 µM Grx2. Subsequently, generated free thiols are selectively modified by addition of PEG5k-maleimide, PEG20k-maleimide, or PEG40k-maleimide (dissolved in water) to a final concentration of 0.8 mM. Thiol alkylation is allowed to proceed for 15 min at room temperature upon quenching with 0.5 mM cysteine. EDTA is added in excess of calcium (20 mM final concentration) and the entire content loaded onto a 1 ml HiTrap Q FF column (Amersham Biosciences, GE Healthcare) equilibrated with buffer A (50 mM HEPES, 100 mM NaCl, 1mM EDTA, pH 7.0) to capture FIX Cys variant. After wash with buffer A, one-step elution of bound protein is performed with buffer B (10 mM GlyGly, 150 mM NaCl, 10 mM CaCl₂, 0.01% Tween 80, pH 7.0) directly onto a HiLoad Superdex 200 16/60 pg column (Amersham Biosciences) mounted in front of the HiTrap column. PEGylated and non-PEGylated species are separated at a flow rate of 1 ml/min and detected by absorption at 280 nm. The peak containing PEGylated FIX Cys variant is collected and stored at -80°C.

**Table 1 DNA oligos used for construction of plasmids.**

| **Primer** | **Plasmid** | **Sequence (5'→3')** |
|---|---|---|
| oHOJ98-f | pHOJ294 | GCCGCCGGCATATGAAGCTATACATTTACGATCACTGCCC |
| oHOJ98-r | pHOJ294 | CCGCCGCCCTCGAGAATCGCCATTGATGATAACAAATTGATTTGTG |
| oHOJ137-f | pHOJ338 | CTATGTAAATTCTACTGAAGCTTGCACCATTTTGGATAACATCAC |
| oHOJ137-r | pHOJ338 | GTGATGTTATCCAAAATGGTGCAAGCTTCAGTAGAATTTACATAG |
| oHOJ155-f | pHOJ358 | GGATTAAGGAAAAAACAAAGCTCACTTGCTAAGCGGCCGCTTCCC |
| oHOJ155-r | pHOJ358 | GGGAAGCGGCCGCTTAGCAAGTGAGCTTTGTTTTTTCCTTAATCC |

### REFERENCES

Begbie M. E., Mamdani A., Gataiance S., Eltringham-Smith L. J., Bhakta V., Hortelano G., and Sheffield W. P. (2005) An important role for the activation peptide domain in controlling factor IX levels in the blood of haemophilia B mice. Thromb Haemost 94, 1138-1147.
Brandstetter H, Bauer M, Huber R, Lollar P, Bode W(1995) X-ray structure of clotting factor IXa: active site and module structure related to Xase activity and hemophilia B. PNAS 92, 9796-9800.
Chau,M.H. and Nelson, J.W. (1991). Direct measurement of the equilibrium between glutathione and dithiothreitol by high performance liquid chromatography. FEBS Lett. 291, 296-298.
Fernandes, A.P. and Holmgren, A. (2004) Glutaredoxins: glutathione-dependent redox enzymes with functions far beyond a simple thioredoxin backup system. Antioxid.Redox.Signal., 6, 63-74
Gilbert,H.F. (1995). Thiol/disulfide exchange equilibria and disulfide bond stability. Methods Enzymol. 251, 8-28.
Grant,C. (2001). MicroReview: Role of the glutathione/glutaredoxin and thioredoxin systems in yeast growth and response to stress conditions. Mol. Microbiol. 39, 533-541
Grimberg,J., Maguire,S., and Belluscio,L. (1989). A simple method for the preparation of plasmid and chromosomal E. coli DNA. Nucleic Acids Res 17, 8893.
Holmgren,A., Åslund,F. (1995) Glutaredoxin, Method Enzymol. 252, 283-292
Hoffman,C.S. and Winston,F. (1987). A ten-minute DNA preparation from yeast efficiently releases autonomous plasmids for transformation of Escherichia coli. Gene 57, 267-272.
Hoffman M. and Monroe D. M., III (2001) A cell-based model of hemostasis. Thromb Haemost 85, 958-965.
Hopfner K.P., Lang A., Karcher A., Sichler K., Kopetzki E., Brandstetter H., Huber R., Bode W., Engh R.A. (1999) Coagulation factor IXa: the relaxed conformation of Tyr99 blocks substrate binding. Structure with Folding & design. 7, 989-96
Loferer,H., Wunderlich,M., Hennecke,H., and Glockshuber,R. (1995). A bacterial thioredoxin-like protein that is exposed to the periplasm has redox properties comparable with those of cytoplasmic thioredoxins. J. Biol. Chem. 270, 26178-26183.
Luikenhuis,S., Perrone,G., Dawes,I.W., and Grant,C.M. (1998). The yeast Saccharomyces cerevisiae contains two glutaredoxin genes that are required for protection against reactive oxygen species. Mol. Biol. Cell 9, 1081-1091
Lundberg,M., Johansson,C., Chandra,J., Enoksson,M., Jacobsson,G., Ljung,J., Johansson,M., and Holmgren,A. (2001). Cloning and expression of a novel human glutaredoxin (Grx2) with mitochondrial and nuclear isoforms. J Biol Chem 276, 26269-26275
McCarthy et al (2002). Pharmacokinetics of recombinant factor IX after intravenous and subcutaneous administration in dogs and cynomolgus monkeys. Thromb Haemost. 87(5):824-30.
Rodriguez-Manzaneque,M.T., Ros,J., Cabiscol,E., Sorribas,A., and Herrero,E. (1999). Grx5 glutaredoxin plays a central role in protection against protein oxidative damage in Saccharomyces cerevisiae. Mol Cell Biol 19, 8180-8190
Oe,T., Ohyagi,T., Naganuma,A. (1998) Determination of γ-glutamylglutathione and other low-molecular-mass thiol compounds by isocratic high-performance liquid chromatography with fluorimetric detection. J. Chrom. B, 708, 285-289
Ostergaard,H., Tachibana,C., and Winther,J.R. (2004). Monitoring disulfide bond formation in the eukaryotic cytosol. J Cell Biol 166, 337-345.
Padilla,C.A., Martinez-Galisteo,E., Barcena,J.A., Spyrou,G., and Holmgren,A. (1995). Purification from placenta, amino acid sequence, structure comparisons and cDNA cloning of human glutaredoxin. Eur J Biochem 227, 27-34
Riddles,P.W., Blakeley,R.L., and Zerner,B. (1979). Ellman's reagent: 5,5'-dithiobis(2-nitrobenzoic acid)--a reexamination. Anal. Biochem. 94, 75-81.
Sambrook,J., Fritsch,E.F., and Maniatis,T. (1989). Molecular Cloning: A Laboratory Manual. (Cold Spring Harbor, NY: Cold Spring Harbor Laboratory).
Schmidt A. E. and Bajaj S. P. (2003) Structure-function relationships in factor IX and factor IXa. Trends Cardiovasc Med 13, 39-45
Takahashi,N. and Creighton,T.E. (1996). On the reactivity and ionization of the active site cysteine residues of Escherichia coli thioredoxin. Biochemistry 35, 8342-8353.
Wang, E.C.W., Hung, S.-H., Cahoon,M., Hedstrom,L. (1997) The role of Cys191-Cys220 disulfide bond in trypsin: new targets for engineering substrate specificity. Protein Engineering, 10, 405-411
Vlamis-Gardikas,A., Aslund,F., Spyrou,G., Bergman,T., and Holmgren,A. (1997). Cloning, overexpression, and characterization of glutaredoxin 2, an atypical glutaredoxin from Escherichia coli. J. Biol. Chem. 272, 11236-11243.
Yang,Y., Jao,S., Nanduri,S., Starke,D.W., Mieyal,J.J., and Qin,J. (1998). Reactivity of the human thioltransferase (glutaredoxin) C7S, C25S, C78S, C82S mutant and NMR solution structure of its glutathionyl mixed disulfide intermediate reflect catalytic specificity. Biochemistry 37, 17145-17156

## Claims

1. FIX analogue with a prolonged circulatory half-life, wherein amino acid residue E162 is substituted for a cysteine amino acid residue conjugated with a chemical group increasing the molecular weight of the FIX polypeptide.

2. FIX analogue with a prolonged circulatory half-life, wherein at least one of the natural amino residues in position 146-180, is substituted for a cysteine amino acid residue conjugated with a chemical group increasing the molecular weight of the FIX polypeptide.

3. FIX analogue according to claim 2, wherein at least one of the natural amino residues selected from positions 155, 156, 157, 158, 159, 160, 161, 162, 163, 164, 167, 168,169, 170, 171, 172, 173, 174, 175, 176 177, is substituted for a cysteine amino acid residue conjugated with a chemical group increasing the molecular weight of the FIX polypeptide.

4. FIX analogue according to claim 3, wherein at least one of the natural amino residues selected form positions 160, 161, 162, 163, 164, 165 and 166, is substituted for a cysteine amino acid residue conjugated with a chemical group increasing the molecular weight of the FIX polypeptide.

5. FIX analogue according to any of the previous claims, wherein the chemical group is selected from polyethylene glycols (PEG).

6. FIX analogue according to claim 5, wherein the polyethyleneglycol has an average molecular weight of in the range of 500-100,000, such as 1000-75,000, or 2,000-60,000.

7. FIX analogue according to any of the previous claims, wherein the analogue has a circulatory half-life of at least 1.5 times that of wild-type FIX.

8. FIX analogue according to any of the previous claims, wherein the analogue, when measured in a clotting assay, has a biological activity of at least 20% of wild-type FIX.

9. A method for preparing a FIX analogue according to any of the previous claims, comprising the steps of a) selectively reducing an engineered FIX polypeptide comprising at least one non-native cysteine in a position selected from positions 146-180 conjugated through a disulfide bridge to a low-molecular weight thiol (RS-CYS), by allowing the low-molecular weight thiol-conjugated FIX to react with a mixture comprising a redox buffer and b) a simultaneous or subsequent step of conjugating at least one of the selectively reduced cysteine (HS-Cys) moieties with a chemical group.

10. A method for preparing a FIX analogue according to any of claims 1-8, comprising the steps of a) selectively reducing an engineered FIX polypeptide comprising at least one non-native cysteine in a position selected from positions 146-180 conjugated through a disulfide bridge to a low-molecular weight thiol (RS-CYS), by allowing the low-molecular weight thiol-conjugated FIX to react with a mixture comprising a triarylphosphine-3,3',3"-trisulfonic acid compound and b) a simultaneous or subsequent step of conjugating at least one of the selectively reduced cysteine (HS-Cys) moieties with a chemical group..

11. The method according to claim 10, wherein the chemical group is a polyethyleneglycol, in particular one having an average molecular weight of in the range of 500-100,000, such as 1000-75,000, or 2,000-60,000.

12. A pharmaceutical formulation for the treatment of a haemophilia patient comprising a therapeutically effective amount of a FIX analogue according to claim 1-8 together with a pharmaceutically acceptable carrier.


SEQUENCE LISTING

<110>  Novo Nordisk A/S

<120>  Prolonged factor IX analogues and derivatives

<130>  7387.215-EP

<160>  1

<170>  PatentIn version 3.3

<210>  1
<211>  415
<212>  PRT
<213>  Homo sapiens


<220>
<221>  The three-letter indication "Xaa" means 4-carboxyglutamic acid
       (gamma-carboxyglutamate)
<222>  (1)..(415)

<400>  1

Tyr Asn Ser Gly Lys Leu Xaa Xaa Phe Val Gln Gly Asn Leu Xaa Arg
1               5                   10                  15


Xaa Cys Met Xaa Xaa Lys Cys Ser Phe Xaa Xaa Ala Arg Xaa Val Phe
            20                  25                  30


Xaa Asn Thr Xaa Arg Thr Thr Xaa Phe Trp Lys Gln Tyr Val Asp Gly
        35                  40                  45


Asp Gln Cys Glu Ser Asn Pro Cys Leu Asn Gly Gly Ser Cys Lys Asp
    50                  55                  60


Asp Ile Asn Ser Tyr Glu Cys Trp Cys Pro Phe Gly Phe Glu Gly Lys
65                  70                  75                  80


Asn Cys Glu Leu Asp Val Thr Cys Asn Ile Lys Asn Gly Arg Cys Glu
                85                  90                  95


Gln Phe Cys Lys Asn Ser Ala Asp Asn Lys Val Val Cys Ser Cys Thr
            100                 105                 110


Glu Gly Tyr Arg Leu Ala Glu Asn Gln Lys Ser Cys Glu Pro Ala Val
        115                 120                 125

Pro Phe Pro Cys Gly Arg Val Ser Val Ser Gln Thr Ser Lys Leu Thr
    130                 135                 140

Arg Ala Glu Thr Val Phe Pro Asp Val Asp Tyr Val Asn Ser Thr Glu
145                 150                 155                 160

Ala Glu Thr Ile Leu Asp Asn Ile Thr Gln Ser Thr Gln Ser Phe Asn
                165                 170                 175

Asp Phe Thr Arg Val Val Gly Gly Glu Asp Ala Lys Pro Gly Gln Phe
            180                 185                 190

Pro Trp Gln Val Val Leu Asn Gly Lys Val Asp Ala Phe Cys Gly Gly
        195                 200                 205

Ser Ile Val Asn Glu Lys Trp Ile Val Thr Ala Ala His Cys Val Glu
    210                 215                 220

Thr Gly Val Lys Ile Thr Val Val Ala Gly Glu His Asn Ile Glu Glu
225                 230                 235                 240

Thr Glu His Thr Glu Gln Lys Arg Asn Val Ile Arg Ile Ile Pro His
                245                 250                 255

His Asn Tyr Asn Ala Ala Ile Asn Lys Tyr Asn His Asp Ile Ala Leu
            260                 265                 270

Leu Glu Leu Asp Glu Pro Leu Val Leu Asn Ser Tyr Val Thr Pro Ile
        275                 280                 285

Cys Ile Ala Asp Lys Glu Tyr Thr Asn Ile Phe Leu Lys Phe Gly Ser
    290                 295                 300

Gly Tyr Val Ser Gly Trp Gly Arg Val Phe His Lys Gly Arg Ser Ala
305                 310                 315                 320

Leu Val Leu Gln Tyr Leu Arg Val Pro Leu Val Asp Arg Ala Thr Cys
                325                 330                 335

Leu Arg Ser Thr Lys Phe Thr Ile Tyr Asn Asn Met Phe Cys Ala Gly
            340                 345                 350

Phe His Glu Gly Gly Arg Asp Ser Cys Gln Gly Asp Ser Gly Gly Pro

355                 360                 365

His Val Thr Glu Val Glu Gly Thr Ser Phe Leu Thr Gly Ile Ile Ser
    370                 375                 380

Trp Gly Glu Glu Cys Ala Met Lys Gly Lys Tyr Gly Ile Tyr Thr Lys
385                 390                 395                 400

Val Ser Arg Tyr Val Asn Trp Ile Lys Glu Lys Thr Lys Leu Thr
                405                 410                 415